(19) Europäisches Patentamt
European Patent Office
Office européen des brevets

(11) EP 2 715 828 B1

(12) **EUROPÄISCHE PATENTSCHRIFT**

(45) Veröffentlichungstag und Bekanntmachung des Hinweises auf die Patenterteilung:
**29.08.2018 Patentblatt 2018/35**

(21) Anmeldenummer: **12721183.7**

(22) Anmeldetag: **16.05.2012**

(51) Int Cl.:
***H01L 51/54*** *(2006.01)* ***C07C 211/43*** *(2006.01)*
***C07D 409/00*** *(2006.01)*

(86) Internationale Anmeldenummer:
**PCT/EP2012/002118**

(87) Internationale Veröffentlichungsnummer:
**WO 2012/163480 (06.12.2012 Gazette 2012/49)**

(54) **ORGANISCHE ELEKTRONISCHE VORRICHTUNG**

ORGANIC ELECTRONIC DEVICE

DISPOSITIF ELECTRONIQUE ORGANIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.05.2011 DE 102011102586**

(43) Veröffentlichungstag der Anmeldung:
**09.04.2014 Patentblatt 2014/15**

(73) Patentinhaber: **Merck Patent GmbH**
**64293 Darmstadt (DE)**

(72) Erfinder:
- **PAN, Junyou**
**60320 Frankfurt am Main (DE)**
- **SCHULTE, Niels**
**65779 Kelkheim (DE)**

(56) Entgegenhaltungen:
**WO-A1-2008/082164 JP-A- 2007 214 364**
**US-A1- 2002 045 061 US-A1- 2002 058 155**

- **HINO Y ET AL: "RED PHOSPHORESCENT ORGANIC LIGHT-EMITTING DIODES USING MIXTURE SYSTEM OF SMALL-MOLECULE AND POLYMER HOST", JAPANESE JOURNAL OF APPLIED PHYSICS, JAPAN SOCIETY OF APPLIED PHYSICS, JP, Bd. 44, Nr. 4B, 1. April 2005 (2005-04-01) , Seiten 2790-2794, XP001245853, ISSN: 0021-4922, DOI: 10.1143/JJAP.44.2790**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft organische elektronische Vorrichtungen, insbesondere organische Elektrolumineszenzvorrichtungen nach Anspruch 1 oder 16, die organische Cyclophane, insbesondere als Matrixmaterialien für fluoreszierende oder phosphoreszierende Emitterverbindungen oder als Ladungstransportmaterialien, insbesondere Elektrontransportmaterialien, enthalten sowie diverse organische Cyclophane nach Anspruch 14.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und insbesondere Lebensdauer. Dies gilt vor allem für OLEDs, die im kürzerwelligen Bereich, beispielsweise grün, emittieren.

Die Eigenschaften phosphoreszierender OLEDs werden nicht nur von den eingesetzten Triplettemittern (oder Quintettemittern) bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden häufig Carbazolderivate, z. B. Bis(carbazolyl)biphenyl, als Matrixmaterialien verwendet. Hier besteht noch Verbesserungsbedarf insbesondere in Bezug auf die Lebensdauer und die Glasübergangstemperatur der Materialien.

Weiterhin werden Ketone (WO 2004/093207), Phosphinoxide und Sulfone (WO 2005/003253) als Matrixmaterialien für phosphoreszierende Emitter verwendet. Insbesondere mit Ketonen werden niedrige Betriebsspannungen und lange Lebensdauern erzielt. Es werden weiterhin Triazinderivate als Matrixmaterialien für phosphoreszierende Emitter verwendet (z. B. gemäß WO 2007/063754 oder WO 2008/056746). Allerdings besteht bei Verwendung dieser Matrixmaterialien ebenso wie bei anderen Matrixmaterialien noch Verbesserungsbedarf, insbesondere in Bezug auf die Effizienz und die Lebensdauer der Vorrichtung.

US 2002/058155 A1 offenbart zyklische tertiäre Aminoverbindungen, beispielsweise die Verbindung (24) auf Seite 6, sowie deren Verwendung in organischen Licht emittierenden Bauteilen.

JP 2007/214364 A offenbart strukturell ähnliche zyklische tertiäre Aminoverbindungen und deren Verwendung in OLEDs.

WO 2008/082164 A1 offenbart strukturell ähnliche zyklische tertiäre Aminoverbindungen und deren Verwendung in OLEDs.

Y. Hino et al. Jpn. J. Appl. Phys. 2005, 44, 2790-2794 offenbart eine OLED enthaltend einen rot emittierenden phosphoreszierenden Emitter sowie Poly(n-vinyl)carbazol und 1,3,5-Tris[4-Diphenylamino)phenylbenzol (TDAPB).

US 2002/045061 A1 offenbart eine organische elektrolumineszierende Vorrichtung enthaltend spezielle Triarylamine enthaltend Carbazolderivate.

Es besteht also insbesondere noch Verbesserungsbedarf bei Matrixmaterialien für phosphoreszierende Emitter, welche gleichzeitig zu hohen Effizienzen, langen Lebensdauern und geringen Betriebsspannungen führen.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Lochtransport-/ Elektronenblockiermaterial bzw. Exzitonenblockiermaterial oder als Elektronentransport- bzw. Lochblockiermaterial. Insbesondere ist es die Aufgabe der vorliegenden Erfindung, Matrixmaterialien und Elektronentransportmaterialien bereitzustellen, welche sich auch für grün und blau phosphoreszierende OLEDs eignen. Weiterhin ist es die Aufgabe der vorliegenden Erfindung, Matrixmaterialien für phosphoreszierende Emitter bereitzustellen

Überraschenderweise wurde gefunden, dass organische Cyclophane der Formel (9) sich sehr gut als Matrixmaterialien für phosphoreszierende Emitterverbindungen und auch als Ladungstransportmaterialien eignen und in dieser Verwendung zu OLEDs führen, welche gleichzeitig hohe Effizienzen und geringe Betriebsspannungen aufweisen.

Die vorliegende Erfindung stellt eine organische Elektrolumineszenzvorrichtung bereit, die in wenigstens einer Schicht eine Cyclophan-Verbindung gemäß der folgenden Formel (9) enthält, wie in Anspruch 1 definiert.

Unter einer organischen Elektrolumineszenzvorrichtung wird eine Vorrichtung verstanden, welche mindestens zwei Elektroden (Anode und Kathode) und mindestens eine emittierende Schicht, welche zwischen der Anode und der Kathode angeordnet ist, enthält, wobei mindestens eine Schicht zwischen der Anode und der Kathode mindestens eine Verbindung der Formel (9) und vorzugsweise eine organische bzw. metallorganische Verbindung als phosphoreszierende Emitterverbindung enthält. Die erfindungsgemäße organische Elektrolumineszenzvorrichtung muss nicht notwendigerweise nur Schichten enthalten, welche aus organischen oder metallorganischen Materialien aufgebaut sind. So ist es auch möglich, dass eine oder mehrere Schichten anorganische Materialien enthalten oder ganz aus anorganischen Materialien aufgebaut sind.

Unter den Begriff "Elektrolumineszenz" subsumiert man ein optisches Phänomen und elektrisches Phänomen, bei dem ein Material Licht als Reaktion auf die Anwendung eines elektrischen Feldes emittiert. Im Kontext dieser Erfindung sind folgende organische Elektrolumineszenzvorrichtungen bevorzugt:

1) "Organic light emitting diode" (OLED) wie von R. Friend et al., in Nature 397, 121-128 (1999) beschrieben ist;
2) "Organic light emitting electrochemical cell" (OLEC) wie von Pei et al., in Science, (1995) Vol269 p1086 beschrieben ist;
3) "Organic light emitting field effect transistor" wie beschrieben von Fabio in Cicoira and Clara Santato, in Adv. Funct. Mater. 2007, 17, 3421-3434, wobei "Source" und "Drain" äquivalent zur Anode und Kathode sind.
4) "Organic light emitting electrochemical transistor" wie von C. Yumusak and N. S. Sariciftci, in Appl. Phys. Lett. 97, 033302 (2010) beschrieben ist, wobei "Source" und "Drain" äquivalent zur Anode und Kathode sind.

Erfindungsgemäß bevorzugt ist die organische elektronische Vorrichtung ein eine organische Elektrolumineszenzvorrichtung, insbesondere ein OLED oder OLEC.

Unter einem mono- oder polycylischen aromatischen Ringsystem versteht man im Sinne dieser Erfindung vorzugsweise ein aromatisches Ringsystem mit 6 bis 60 Kohlenstoffatomen, bevorzugt 6 bis 30, besonders bevorzugt 6 bis 10 Kohlenstoffatomen. Unter einem aromatischen Ringsystem im Sinne der vorliegenden Erfindung soll ein System verstanden werden, das nicht notwendigerweise nur aromatische Gruppen enthält, sondern in dem auch mehrere aromatische durch eine kurze nicht-aromatische Einheit (< 10% der von H verschiedenen Atome, vorzugsweise < 5% der von H verschiedenen Atome), wie beispielsweise $sp^3$-hybridisierter C, O, N, etc., oder einer -C(O)- Gruppe unterbrochen sein können. Diese aromatischen Ringsysteme können monocyclisch oder polycyclisch sein, d.h. sie können einen Ring (z.B. Phenyl) oder zwei oder mehr Ringe aufweisen, welche auch kondensiert (z.B. Naphthyl) oder kovalent verknüpft sein können (z.B. Biphenyl), oder eine Kombination von kondensierten und verknüpften Ringen beinhalten.

Bevorzugte aromatische Ringsysteme sind z.B. Phenyl, Biphenyl, Triphenyl, Naphthyl, Anthracyl, Binaphthyl, Phenanthryl, Dihydrophenanthryl, Pyren, Dihydropyren, Chrysen, Perylen, Tetracen, Pentacen, Benzpyren, Fluoren und Inden.

Unter einem mono- oder polycylischen heteroaromatischen Ringsystem versteht man im Sinne dieser Erfindung vorzugsweise ein heteroaromatisches Ringsystem mit 5 bis 60 Ringatomen, bevorzugt 5 bis 30, besonders bevorzugt 5 bis 14 Ringatomen. Das heteroaromatische Ringsystem enthält mindestens ein Heteroatom ausgewählt aus N, O und S (verbleibenden Atome sind Kohlenstoff). Unter einem heteroaromatischen Ringsystem soll zudem ein System verstanden werden, das nicht notwendigerweise nur aromatische oder heteroaromatische Gruppen enthält, sondern in dem auch mehrere aromatische bzw. heteroaromatische Gruppen durch eine kurze nicht-aromatische Einheit (< 10% der von H verschiedenen Atome, vorzugsweise < 5% der von H verschiedenen Atome), wie beispielsweise $sp^3$-hybridisierter C, O, N, etc., oder einer - C(O)- Gruppe unterbrochen sein können. Diese heteroaromatischen Ringsysteme können monocyclisch oder polycyclisch sein, d.h. sie können einen Ring (z.B. Pyridyl) oder zwei oder mehr Ringe aufweisen, welche auch kondensiert oder kovalent verknüpft sein können, oder eine Kombination von kondensierten und verknüpften Ringen beinhalten.

Bevorzugte heteroaromatische Ringsysteme sind z.B. 5-gliedrige Ringe wie Pyrrol, Pyrazol, Imidazol, 1,2,3-Triazol, 1,2,4-Triazol, Tetrazol, Furan, Thiophen, Selenophen, Oxazol, Isoxazol, 1,2-Thiazol, 1,3-Thiazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 6-gliedrige Ringe wie Pyridin, Pyridazin, Pyrimidin, Pyrazin, 1,3,5-Triazin, 1,2,4-Triazin, 1,2,3-Triazin, 1,2,4,5-Tetrazin, 1,2,3,4-Tetrazin, 1,2,3,5-Tetrazin, oder kondensierte Gruppen wie Indol, Isoindol, Indolizin, Indazol, Benzimidazol, Benzotriazol, Purin, Naphthimidazol, Phenanthrimidazol, Pyridimidazol, Pyrazinimidazol, Chinoxalinimidazol, Benzoxazol, Naphthoxazol, Anthroxazol, Phenanthroxazol, Isoxazol, Benzothiazol, Benzofuran, Isobenzofuran, Dibenzofuran, Chinolin, Isochinolin, Pteridin, Benzo-5,6-chinolin, Benzo-6,7-chinolin, Benzo-7,8-chinolin, Benzoisochinolin, Acridin, Phenothiazin, Phenoxazin, Benzopyridazin, Benzopyrimidin, Chinoxalin, Phenazin, Naphthyridin, Azacarbazol, Benzocarbolin, Phenanthridin, Phenanthrolin, Thieno[2,3b]thiophen, Thieno[3,2b]thiophen, Dithienothiophen, Isobenzothiophen, Dibenzo-thiophen, Benzothiadiazothiophen oder Kombinationen dieser Gruppen. Besonders bevorzugt sind Imidazol, Benzimidazol und Pyridin.

Ist das monocyclische oder polycyclische aromatische oder heteroaromatische Ringsystem wie im Fall von $Ar_1$ ein bivalentes System, so finden die zwei Bindungen zu den Stickstoffatomen der Verbindung der Formel (1) vorzugsweise an einem aromatischen Cyclus statt. Es ist des Weiteren bevorzugt, dass die zwei Bindungen zu den Stickstoffatomen der Formel (1) über Positionen des einen aromatischen Cyclus in der Weise stattfinden, dass sie beide in meta-Position zueinander stehen. In anderen Worten findet die Verknüpfung zu den Ringstickstoffatomen der Verbindung der

Formel (1) in den Positionen 1 und 3 eines aromatischen Ringes statt.

Ist das monocyclische oder polycyclische aromatische oder heteroaromatische Ringsystem wie im Fall von $Ar_2$ ein monovalenter Rest, so findet die Bindung vorzugsweise über ein aromatisches Atom des Ringsystems statt.

Die Ringsysteme $Ar^1$ und $Ar^2$ können unsubstituiert oder substituiert vorliegen. Liegen sie substituiert vor, so können sie einen oder mehrere Reste $R^1$ aufweisen. $R^1$ ist bei jedem Auftreten gleich oder verschieden aus der Gruppe ausgewählt, die aus folgendem besteht: H, D, F, Cl, Br, I, CHO, $N(Ar)_2$, C(=O)Ar, $P(=O)(Ar)_2$, S(=O)Ar, $S(=O)_2Ar$, $CR^2=CR^2Ar$, CN, $NO_2$, $Si(R^2)_3$, $B(OR^2)_2$, $B(R^2)_2$, $B(N(R^2)_2)_2$, $OSO_2R^2$, eine Alkyl-, Alkoxy- oder Thioalkoxygruppe, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch F, Cl, Br, I, CN oder $NO_2$ ersetzt sein können, oder ein aromatisches oder heteroaromatisches Ringsystem, das jeweils durch einen oder mehrere Reste $R^2$ substituiert sein kann, oder eine Aryloxy- oder Heteroaryloxygruppe, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches, aromatisches oder heteroaromatisches Ringsystem bilden; wobei Ar bei jedem Auftreten gleich oder verschieden ein mono- oder polycyclisches aromatisches oder heteroaromatisches Ringsystem, das mit einem oder mehreren Resten $R^2$ substituiert sein kann, ist; dabei können auch zwei Reste Ar, welche an dasselbe Stickstoff-, Phosphor- oder Boratom binden, durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verknüpft sein; und wobei $R^2$ bei jedem Auftreten gleich oder verschieden H, D oder ein aliphatischer, aromatischer und/oder heteroaromatischer Kohlenwasserstoffrest ist, in dem auch H-Atome durch F ersetzt sein können; dabei können zwei oder mehrere benachbarte Substituenten $R^2$ auch miteinander ein mono- oder polycyclisches, aliphatisches oder aromatisches Ringsystem bilden;

Im Rahmen der vorliegenden Erfindung werden unter einer Alkylgruppe, lineare, verzweigte oder cyclische Alkylgruppen verstanden. Die linearen Alkylgruppen haben vorzugsweise 1 bis 6, 1 bis 10 oder 1 bis 40 Kohlenstoffatome. Die verzweigten oder cyclischen Alkylgruppen haben vorzugsweise 3 bis 6, 3 bis 10 oder 3 bis 40 Kohlenstoffatome. Bevorzugt sind in allen drei Fällen Alkylgruppen mit 1 bis 6 Kohlenstoffatomen, besonders bevorzugt 1 bis 3 Kohlenstoffatomen. Ein oder mehrere Wasserstoffatome an diesen Alkylgruppen können vorzugsweise auch durch ein Fluoratom ersetzt sein. Außerdem können ein oder mehrere der $CH_2$-Gruppen dieser Einheiten durch NR ersetzt sein (R ist dabei ein Rest, der aus der Gruppe ausgewählt ist, die aus H und $C_{1-6}$-Alkyl besteht). Wenn eine oder mehrere der $CH_2$-Gruppen durch NR ersetzt ist, ist es besonders bevorzugt, dass nur eine dieser Gruppen ersetzt ist. Die Alkylgruppen können auch Alkenyl- oder Alkinyl-Gruppen, d.h. Gruppen mit eine oder mehreren Doppel- oder Dreifachbindungen. Beispiele solcher Verbindungen schließen die folgenden ein: Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl und 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl.

Unter einer Alkoxygruppe bzw. Thioalkoxygruppe versteht man eine wie oben definierte Alkylgruppe, die über eine O- oder S-Atom gebunden ist.

Bevorzugte Alkoxygruppen sind Methoxy, Trifluormethoxy, Ethoxy, n-Propoxy, i-Propoxy, n-Butoxy, i-Butoxy, s-Butoxy, t-Butoxy oder 2-Methylbutoxy.

Erfindungsgemäße aliphatische Kohlenwasserstoffreste sind vorzugsweise lineare bzw. verzweigte oder cyclische Alkylgruppen, Alkenylgruppen oder Alkinylgruppen mit vorzugsweise 1 bis 20 bzw. 3 bis 20 Kohelnstoffatomen, bei denen ein oder mehr Kohlenstoffatome durch O, N oder S ersetzt sein können. Zudem kann ein oder mehrere Wasserstoffatome durch Fluor ersetzt sein. Beispiele der aliphatischen Kohlenwasserstoffe mit 1 bis 20 Kohlenstoffatomen schließen die folgenden ein: Methyl, Ethyl, n-Propyl, isoPropyl, n-Butyl, iso-Butyl, sec-Butyl (1-Methylpropyl), tert-Butyl, iso-Pentyl, n-Pentyl, tert-Pentyl (1,1- Dimethylpropyl), 1,2-Dimethylpropyl, 2,2-Dimethylpropyl (neopentyl), 1-Ethylpropyl, 2-Methylbutyl, n-Hexyl, isoHexyl, 1,2-Dimethylbutyl, 1-Ethyl-1-methylpropyl, 1-Ethyl-2-methylpropyl, 1,1,2-Trimethylpropyl, 1,2,2-Trimethylpropyl, 1-Ethylbutyl, 1-Methylbutyl, 1,1-Dimethylbutyl, 2,2-Dimethylbutyl, 1,3-Dimethylbutyl, 2,3-Dimethylbutyl, 3,3-Dimethylbutyl, 2-Ethylbutyl, 1-Methylpentyl, 2-Methylpentyl, 3-Methylpentyl, Cyclopentyl, Cyclohexyl, Cycloheptyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl und Octinyl.

Ein aromatischer oder heteroaromatischer Kohlenwasserstoffrest kann mono- oder polycyclisch sein und enthält vorzugsweise 5 bis 20, stärker bevorzugt 5 bis 10, am bevorzugtesten 5 oder 6 aromatische Ringatome. Ist die Einheit eine aromatische Einheit, so enthält sie bevorzugt 6 bis 20, ganz bevorzugt 6 bis 10, ganz besonders bevorzugt 6 Kohlenstoffatome als Ringatome. Ist die Einheit eine heteroaromatische Einheit enthält sie 5 bis 20, bevorzugt 5 bis 10, ganz bevorzugt 5 aromatische Ringatome, von denen mindestens eines ein Heteroatom ist. Die Heteroatome sind bevorzugt ausgewählt aus N, O und/oder S. Dabei wird unter einer aromatischen bzw. heteroaromatischen Einheit

entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, Benzothiophen, Benzofuran und Indol etc., verstanden.

Erfindungsgemäße Beispiele für den aromatischen oder heteroaromatischen Kohlenwasserstoffrest sind demgemäß: Benzol, Naphthalin, Anthracen, Phenanthren, Pyren, Chrysen, Benzanthracen, Perylen, Naphthacen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxy- oder Heteroaryloxygruppe versteht man einen aromatischen oder heteroaromatischen Kohlenwasserstoffrest wie oben definiert, der über ein Sauerstoffatom gebunden ist.

Unter einem aliphatischen Ringsystem versteht man ein mono- oder polycyclisches Ringsystem aus vorzugsweise 4 bis 30 $CH_2$-Einheiten (in Falle von polycyclisch auch CH-Einheiten), bevorzugt 5 bis 20 $CH_2$-Einheiten, besonders bevorzugt 5 Ringatomen, das bis zu drei, bevorzugt bis zu 2, bevorzugt 2 Heteroatome ausgewählt aus N, O, S, vorzugsweise N, enthalten kann. Erfindungsgemäß bevorzugte Beispiele sind 1,2-Diazocyclopentan bzw. bevorzugt 1,3-Diazocyclopentan.

Die erfindungsgemäßen organischen elektronischen Vorrichtungen können für verschiedene Anwendungen verwendet werden, beispielsweise für einfarbige oder mehrfarbige Displays, für Beleuchtungsanwendungen oder für medizinische oder kosmetische Anwendungen, beispielsweise in der Phototherapie.

Die bevorzugte organische elektronische Vorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierenden Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss.

Dabei kann die organische elektronische Vorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Dabei ist es möglich, dass alle emittierenden Schichten fluoreszierende Schichten sind oder dass alle emittierenden Schichten phosphoreszierende Schichten sind oder dass eine oder mehrere emittierende Schicht(en) (eine) fluoreszierende Schicht(en) und eine oder mehrere andere Schicht(en) (eine) phosphoreszierende Schicht(en) sind.

Die erfindungsgemäße organische elektronische Vorrichtung kann dabei unterschiedliche Schichten enthalten, worin in mindestens einer Schicht eine Verbindung gemäß Formel (9) eingesetzt wird, je nach Verwendungszweck und Anwendungsgebiet.

In dieser Erfindung ist eine organische elektronische Vorrichtung bevorzugt, die in einer emittierenden Schicht eine Verbindung gemäß Formel (9) als Matrixmaterial für fluoreszierende oder phosphoreszierende Emitter, insbesondere für phosphoreszierende Emitter, und/oder in einer Lochblockierschicht als Lochblockiermaterial und/oder in einer Elektronentransportschicht als Elektronentransportmaterial bzw. in exzitonenblockierenden Schicht als exzitonenblockierenden Materials und/oder in einer Lochtransportschicht als Lochtranssportmaterial, je nach Verwendungszweck und Anwendungsgebiet, enthält.

In einer weiteren Ausführungsform der Erfindung ist die erfindungsgemäße organische elektronische Vorrichtung besonders bevorzugt eine, worin die Verbindung gemäß Formel (9) als Matrixmaterial für eine fluoreszierende oder phosphoreszierende Verbindung, insbesondere für eine phosphoreszierende Verbindung, in einer emittierenden Schicht eingesetzt wird. Dabei kann die organische elektronische Vorrichtung eine emittierende Schicht enthalten, oder sie kann mehrere emittierende Schichten enthalten, wobei mindestens eine emittierende Schicht mindestens eine Verbindung gemäß Formel (9) als Matrixmaterial enthält.

Die geeignete und bevorzugte Ausführungsform für Verbindungen als Matrixmaterial wird wie folgt erläutert.

Die Verbindungen gemäß Formeln (9), die als Matrixmaterial geeignet sind, sind dadurch gekennzeichnet,

dass in Formel (9) wenigstens ein Vertreter der $Ar^1$ und/oder $Ar^2$ ein heteroaromatisches Ringsystem darstellt.

Die erfindungsgemäße organische elektronische Vorrichtung umfasst vorzugsweise eine Verbindung gemäß Formel (9) in einer emittierenden Schicht als Matrixmaterial, worin wenigstens ein Vertreter der $Ar^1$ eine bivalente Gruppe gemäß einer der folgenden Formeln (2) bis (8) enthält:

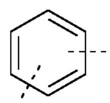
Formel (2)

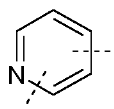
Formel (3)

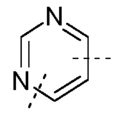
Formel (4)

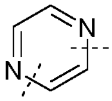
Formel (5)

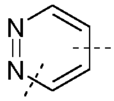
Formel (6)

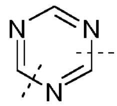
Formel (7)

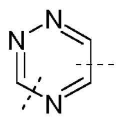
Formel (8)

wobei die gestrichelten Linien die Bindungen zu den Ringstickstoffatomen der Verbindung der Formel (9) darstellen. Sie stehen vorzugsweise zueinander in meta-Position, d.h. in den Positionen 1 und 3 des aromatischen Ringes zueinander.

Insbesondere wenn alle $Ar^2$ aromatische Ringsystem ohne Heteroatome sind, ist nach Anspruch 1 mindestens ein Vertreter der X oder Y in $Ar^1$ gleich N.

Im folgenden beschreiben sind Verbindungen gemäß der Formel (9):

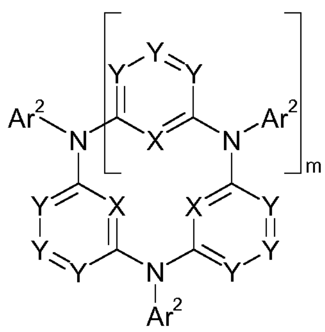
Formel (9),

wobei X und Y gleich $CR^1$ oder N, und m eine ganze Zahl von 1 bis 6, vorzugsweise 1, 2 oder 4 ist, mit der Maßgabe, dass entweder 1.) mindestens ein Vertreter der X oder Y gleich N ist, oder 2.) ein Vertreter der $Ar^2$ ein heteroaromatisches, oder ein aromatisches Ringsystem darstellt, und $R^1$ die gleiche Bedeutung wie in Anspruch 1 definiert aufweist.

Beispiele für besonders bevorzugte Verbindungen gemäß der Formel (9) sind die im Folgenden aufgeführten Verbindungen der Formeln (10) bis (99).

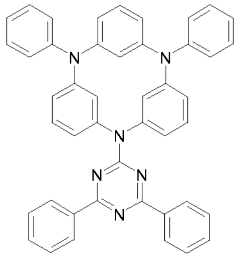

Formel (10)

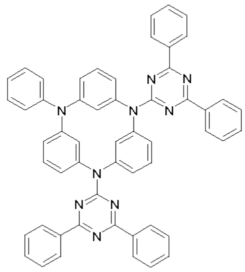

Formel (11)

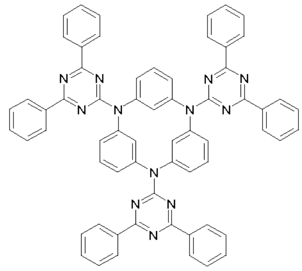

Formel (12)

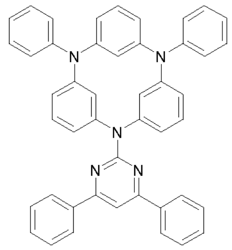

Formel (13)

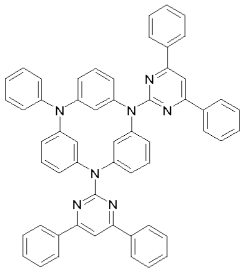

Formel (14)

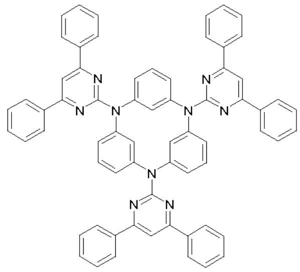

Formel (15)

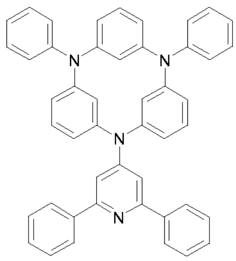

Formel (16)

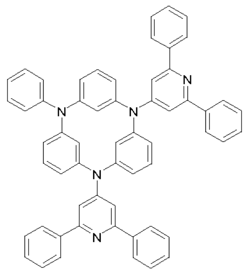

Formel (17)

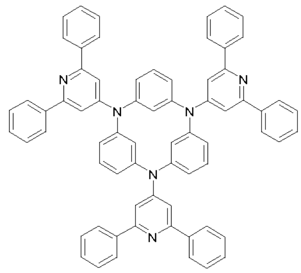

Formel (18)

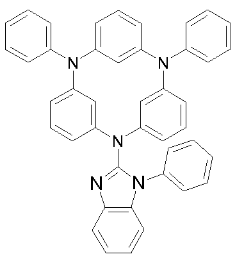

Formel (19)

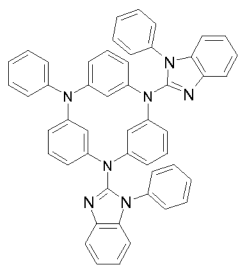

Formel (20)

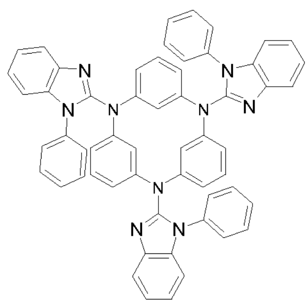

Formel (21)

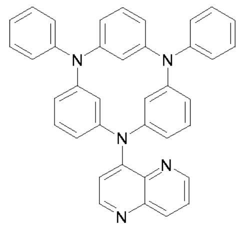

Formel (25)

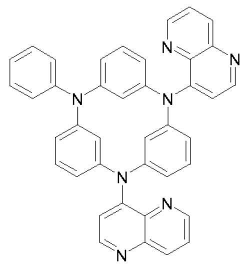

Formel (26)

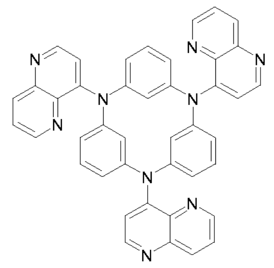

Formel (27)

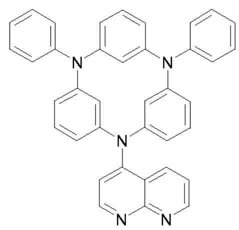

Formel (28)

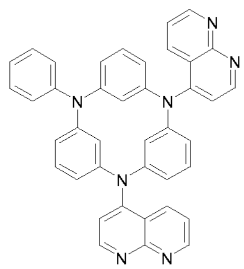

Formel (29)

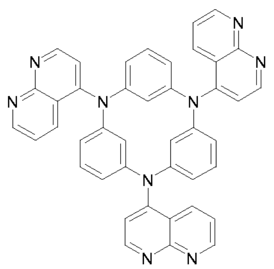

Formel (30)

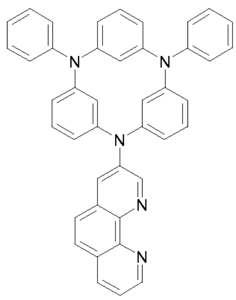

Formel (31)

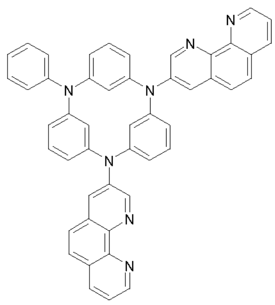

Formel (32)

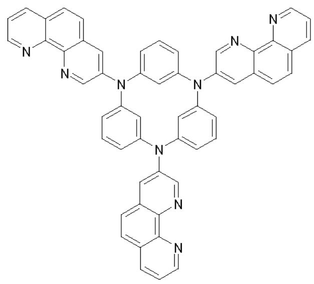

Formel (33)

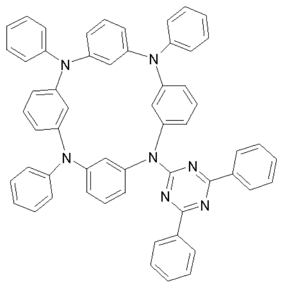

Formel (37)

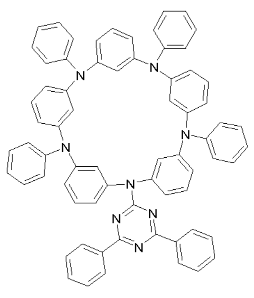

Formel (38)

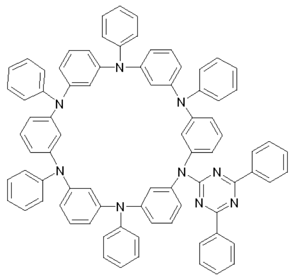

Formel (39)

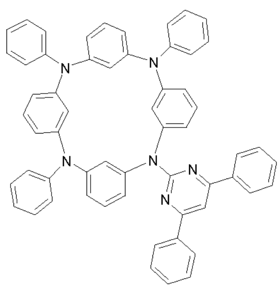

Formel (40)

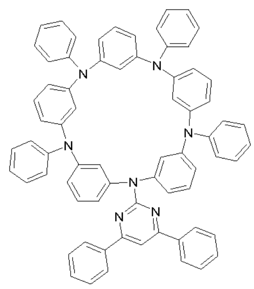

Formel (41)

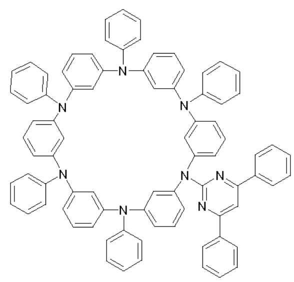

Formel (42)

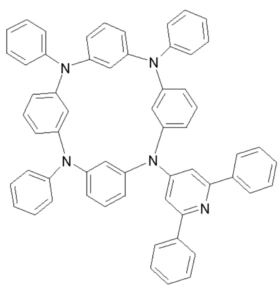

Formel (43)

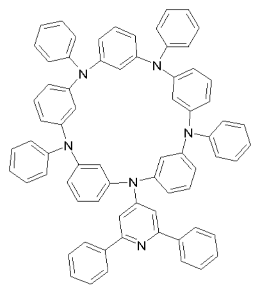

Formel (44)

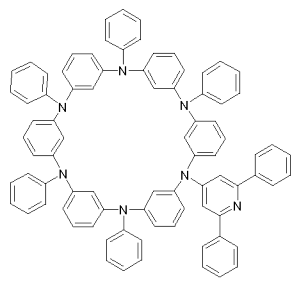

Formel (45)

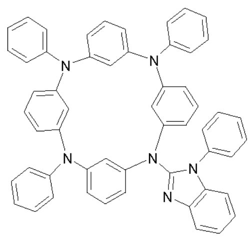

Formel (46)

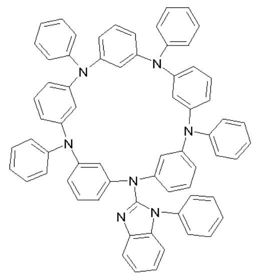

Formel (47)

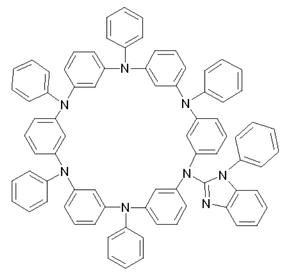

Formel (48)

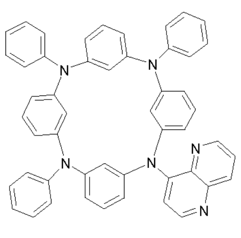

Formel (52)

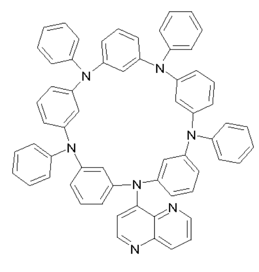

Formel (53)

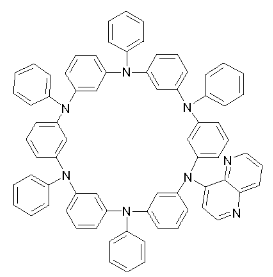

Formel (54)

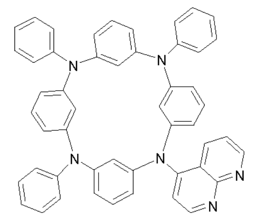

Formel (55)

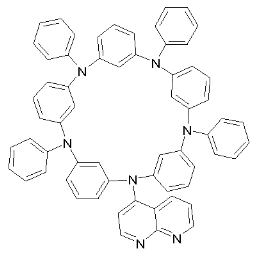

Formel (56)

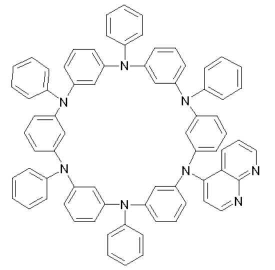

Formel (57)

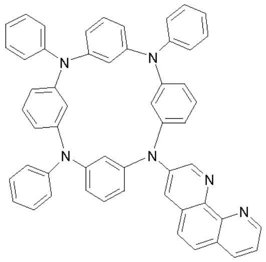

Formel (58)

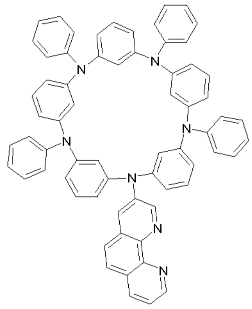

Formel (59)

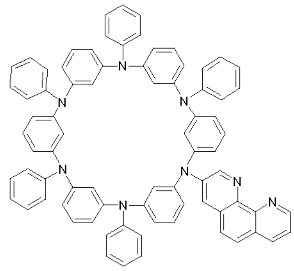

Formel (60)

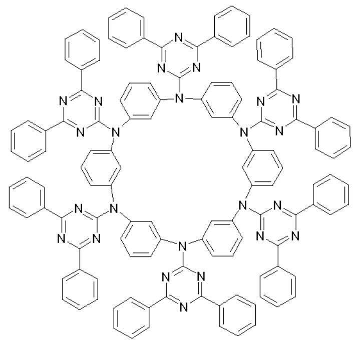

Formel (61)

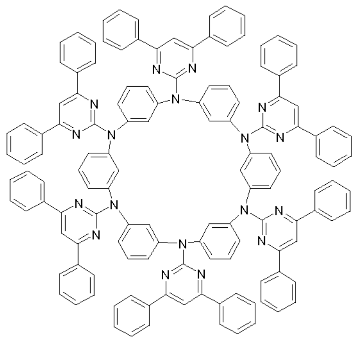

Formel (62)

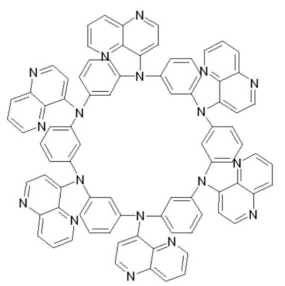

Formel (64)

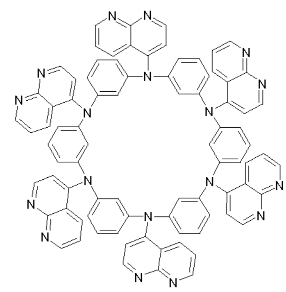

Formel (65)

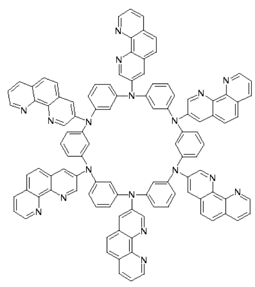

Formel (66)

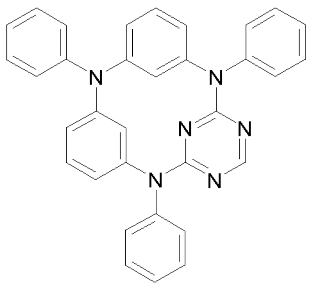

Formel (67)

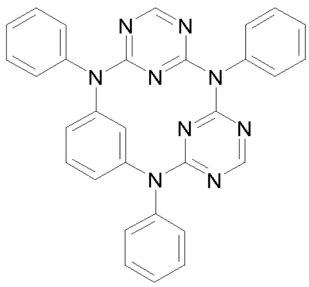

Formel (68)

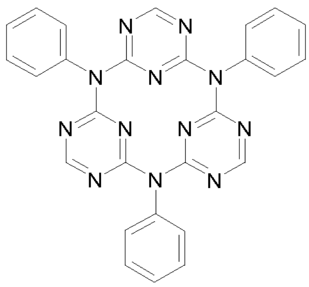

Formel (69)

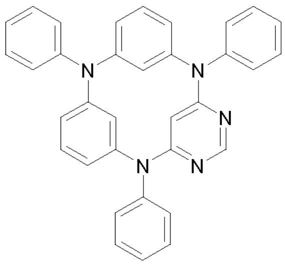

Formel (70)

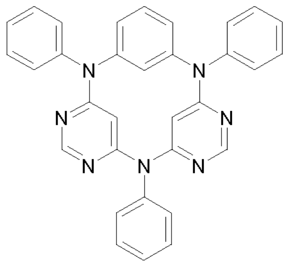

Formel (71)

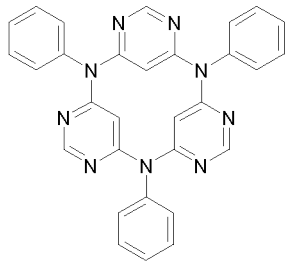

Formel (72)

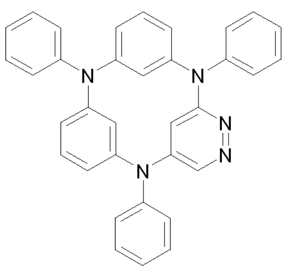

Formel (73)

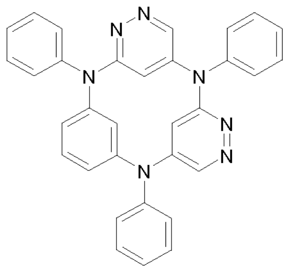

Formel (74)

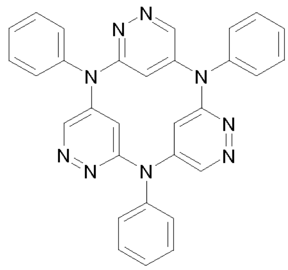

Formel (75)

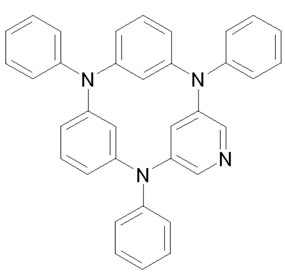

Formel (76)

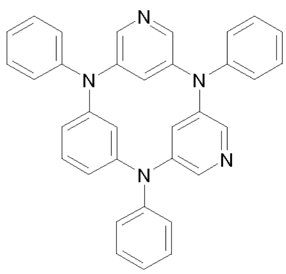

Formel (77)

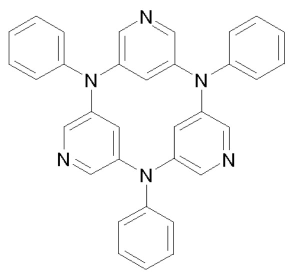

Formel (78)

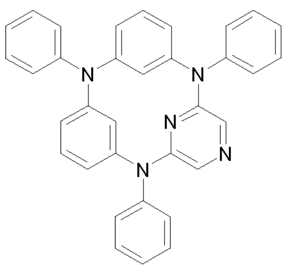

Formel (79)

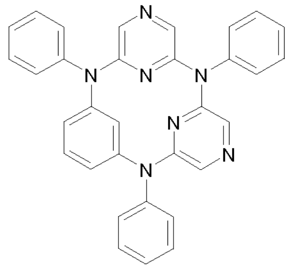

Formel (80)

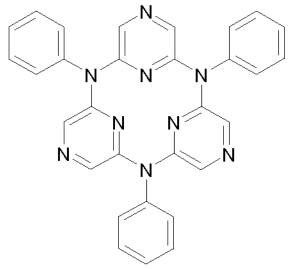

Formel (81)

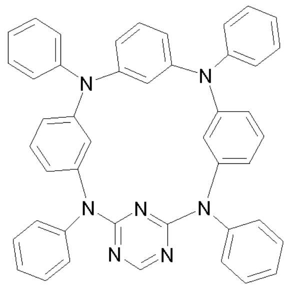

Formel (82)

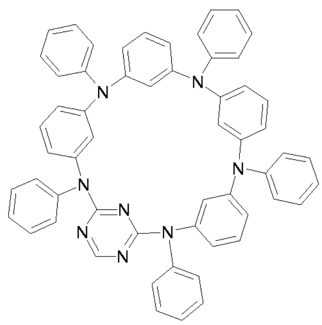

Formel (83)

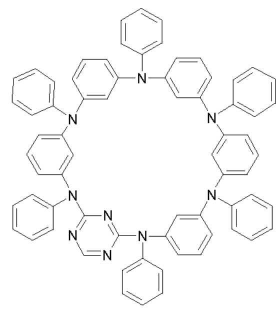

Formel (84)

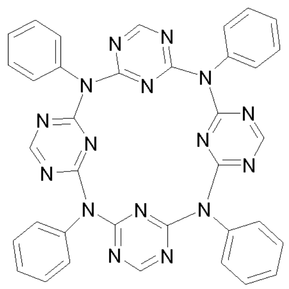

Formel (85)

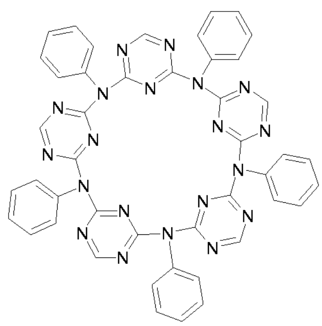

Formel (86)

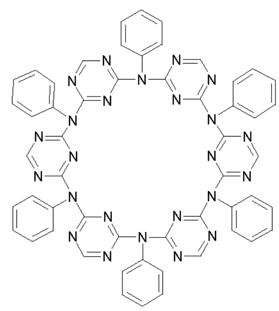

Formel (87)

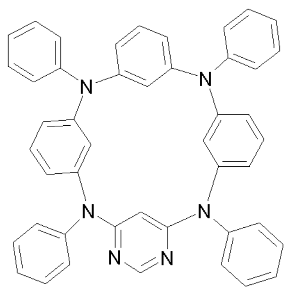

Formel (88)

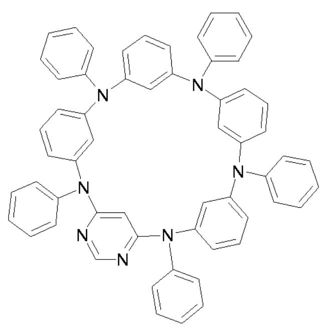

Formel (89)

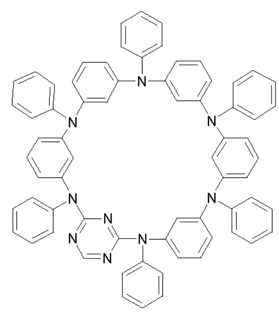

Formel (90)

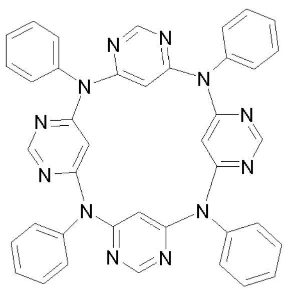
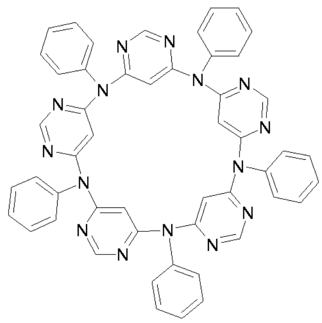
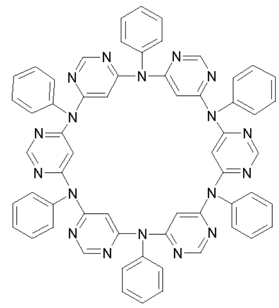

Formel (91) Formel (92) Formel (93)

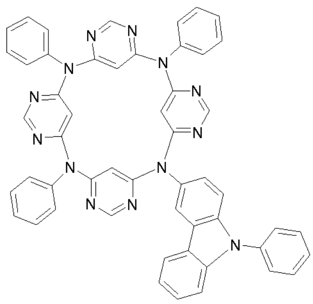
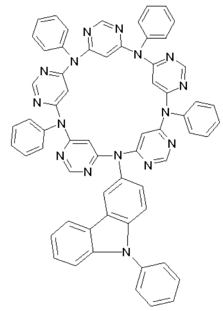
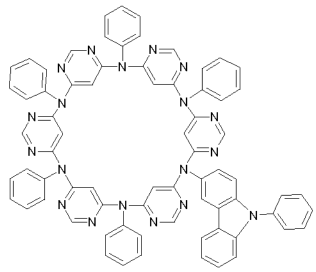

Formel (94) Formel (95) Formel (96)

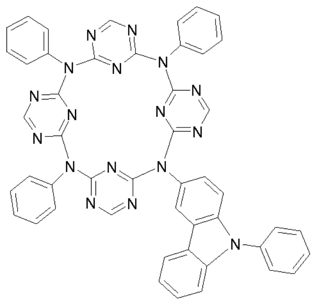
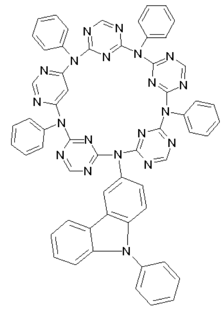
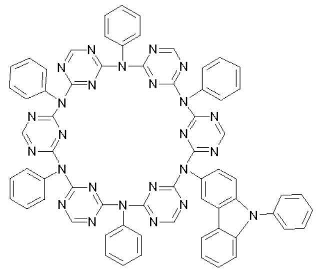

Formel (97) Formel (98) Formel (99).

Beispiele für alternative Verbindungen sind die im Folgenden aufgeführten Verbindungen der Formeln (111) bis (143).

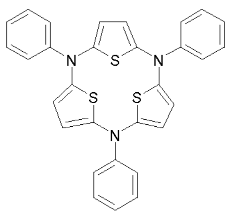
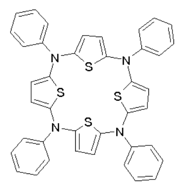
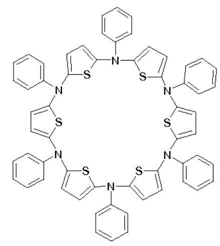

Formel (111) Formel (112) Formel (113)

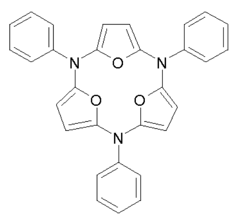

Formel (114)

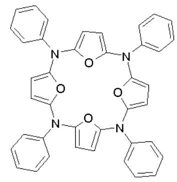

Formel (115)

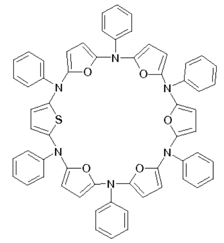

Formel (116)

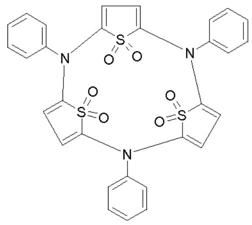

Formel (117)

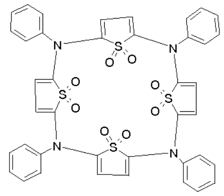

Formel (118)

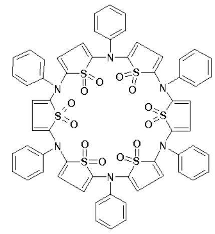

Formel (119)

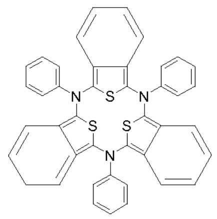

Formel (120)

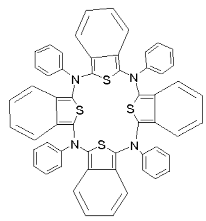

Formel (121)

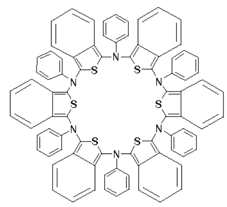

Formel (122)

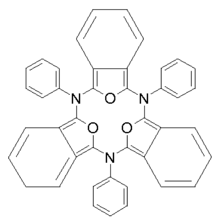

Formel (123)

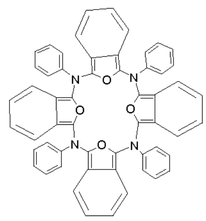

Formel (124)

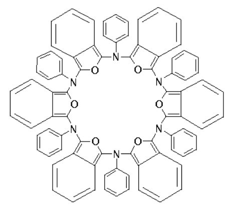

Formel (125)

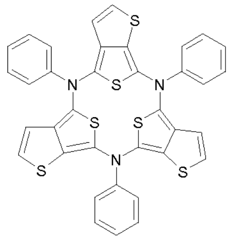

Formel (126)

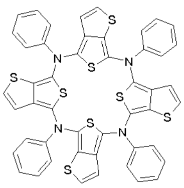

Formel (127)

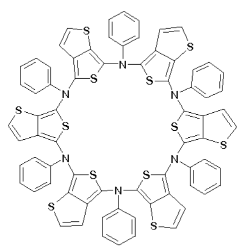

Formel (128)

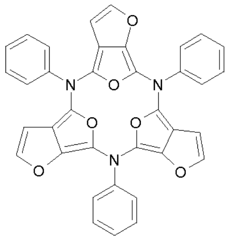

Formel (129)

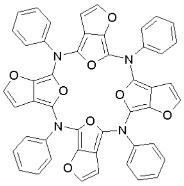

Formel (130)

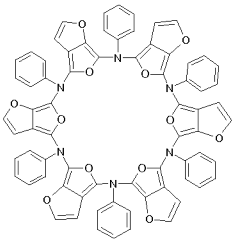

Formel (131)

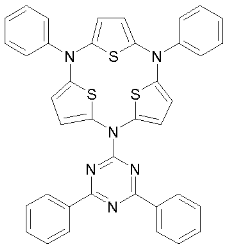

Formel (132)

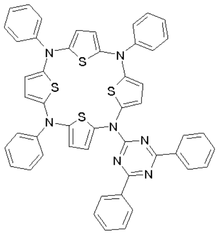

Formel (133)

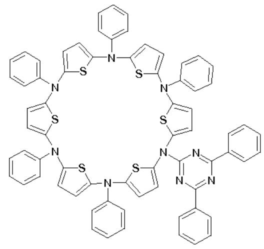

Formel (134)

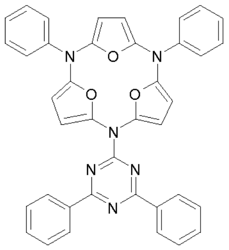

Formel (135)

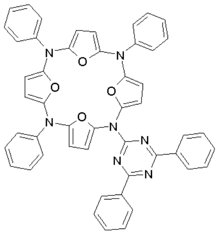

Formel (136)

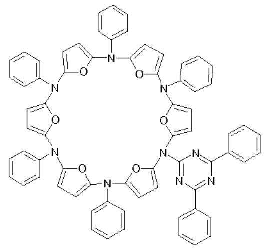

Formel (137)

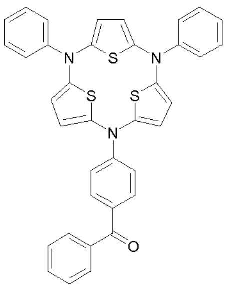

Formel (138)

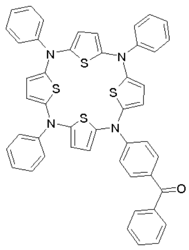

Formel (139)

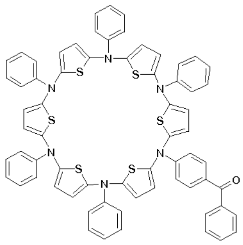

Formel (140)

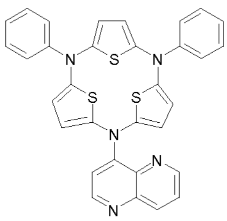

Formel (141)

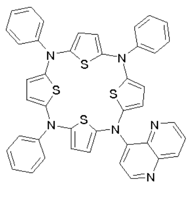

Formel (142)

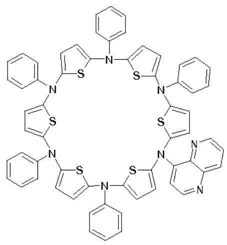

Formel (143).

Es ist in der organischen elektronischen Vorrichtung nach einer der Ausführungsformen der vorliegenden Erfindung bevorzugt, dass, wenigstens ein $Ar^2$ ein monovalenter Rest ist, der aus der Gruppe ausgewählt ist, die aus den Verbindungen der unten stehenden Formeln (144) bis (156) besteht. Dies ist insbesondere dann der Fall, wenn alle Reste $Ar^1$ bivalente aromatische Einheiten sind, die keine Heteroatome enthalten, und wenn die Verbindung als Matrixmaterial und/oder als Elektronentransportmaterial und/oder als Lochblockiermaterial und/oder als Exzitonenblockiermaterterial eingesetzt wird, wie oben und unten beschrieben wird.

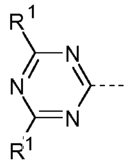
Formel (144)

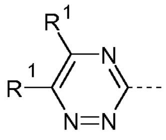
Formel (145)

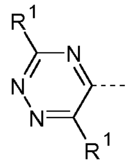
Formel (146)

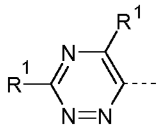
Formel (147)

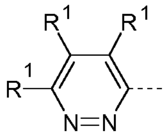
Formel (148)

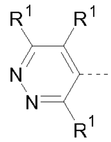
Formel (149)

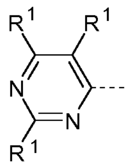
Formel (150)

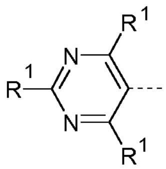
Formel (151)

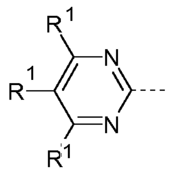
Formel (152)

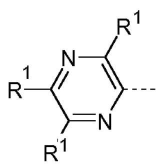
Formel (153)

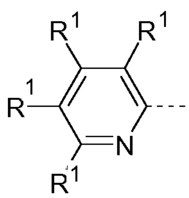
Formel (154)

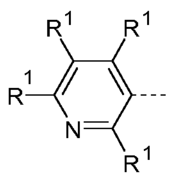
Formel (155)

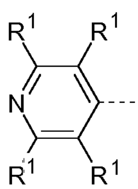
Formel (156),

wobei die gestrichelte Linie jeweils eine Bindung zum Ringstickstoffatom der Verbindung der Formel (9) darstellt; und $R^1$ wie oben definiert ist.

Bevorzugt weisen die Verbindungen gemäß der Formel (9) eine Glasübergangstemperatur $T_G$ von größer als 70°C auf, besonders bevorzugt größer als 90°C, ganz besonders bevorzugt größer als 110°C.

Wie oben beschrieben, werden die Verbindungen gemäß der Formel (9) als Matrixmaterialien für phosphoreszierende Emitterverbindungen verwendet. Dabei werden die phosphoreszierenden Emitterverbindungen vorzugsweise

in der wenigstens einen Schicht der erfindungsgemäßen organischen elektronischen Vorrichtung eingesetzt.

Unter einer phosphoreszierenden Emitterverbindung wird allgemein eine Verbindung verstanden, die Lumineszenz aus einem angeregten Zustand mit höherer Spinmultiplizität zeigt, also ein Spinzustand > 1, wie beispielsweise aus einem angeregten Triplett-Zustand (Triplett-Emitter), aus einem MLCT-Mischzustand oder einem Quintett-Zustand (Quintett-Emitter). Als phosphoreszierende Emitterverbindungen eignen sich insbesondere Verbindungen, die bei geeigneter Anregung Licht, vorzugsweise im sichtbaren Bereich, emittieren und außerdem mindestens ein Atom der Ordnungszahlen > 38 und < 84, besonders bevorzugt > 56 und < 80 enthalten. Bevorzugt werden als Phosphoreszenz-Emitter Verbindungen, die Kupfer, Molybdän, Wolfram, Rhenium, Ruthenium, Osmium, Rhodium, Iridium, Palladium, Platin, Silber, Gold oder Europium enthalten, insbesondere Verbindungen, die Iridium, Platin oder Kupfer enthalten. Beispiele der oben beschriebenen Emitter können den Anmeldungen WO 00/7065, WO 01/41512, WO 02/02714, WO 02/15645, EP 1191613, EP 1191612, EP 1191614, WO 2005/033244 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind.

Als phosphoreszierende Emitterverbindungen eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Besonders bevorzugte organische elektronische Vorrichtungen enthalten als phosphoreszierende Emitterverbindungen mindestens eine Verbindung der Formeln (157) bis (160),

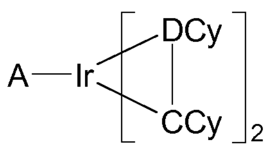

Formel (157)

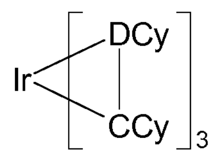

Formel (158)

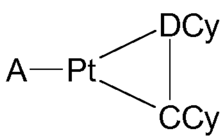

Formel (159)

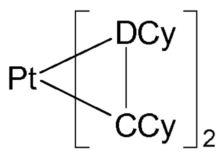

Formel (160)

wobei gilt:

DCy ist gleich oder verschieden bei jedem Auftreten eine cyclische Gruppe, die mindestens ein Donoratom, bevorzugt Stickstoff, Kohlenstoff in Form eines Carbens oder Phosphor, enthält, über welches die cyclische Gruppe an das Metall gebunden ist, und die wiederum einen oder mehrere Substituenten $R^1$ tragen kann; die Gruppen DCy und CCy sind über eine kovalente Bindung miteinander verbunden;

CCy ist gleich oder verschieden bei jedem Auftreten eine cyclische Gruppe, die ein Kohlenstoffatom enthält, über welches die cyclische Gruppe an das Metall gebunden ist und die wiederum einen oder mehrere Substituenten $R^1$ tragen kann;

A ist gleich oder verschieden bei jedem Auftreten ein mono-anionischer, zweizähnig chelatisierender Ligand, bevorzugt ein Diketonatligand.

Dabei kann durch Bildung von Ringsystemen zwischen mehreren Resten $R^1$ auch eine Brücke zwischen den Gruppen DCy und CCy vorliegen.

Beispiele der oben beschriebenen Emitter können den Anmeldungen WO 00/70655, WO 01/41512, WO 02/02714, WO 02/15645, EP 1191613, EP 1191612, EP 1191614 und WO 2005/033244 entnommen werden. Generell

eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden. Beispiele für geeignete phosphoreszierende Verbindungen sind in der folgenden Tabelle aufgeführt.

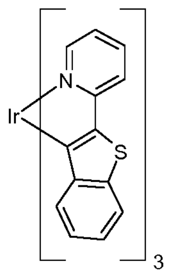

Formel (161)

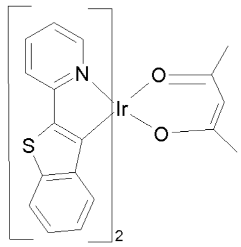

Formel (162)

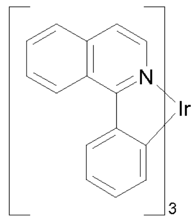

Formel (163)

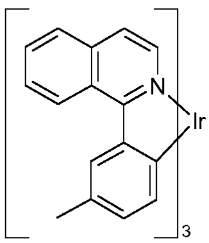

Formel (164)

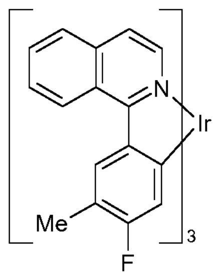

Formel (165)

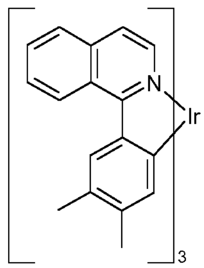

Formel (166)

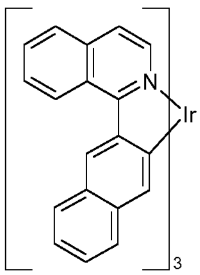

Formel (167)

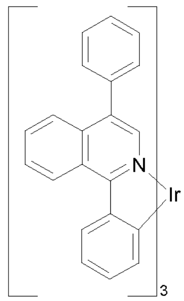

Formel (168)

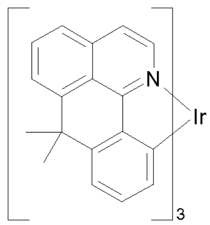

Formel (169)

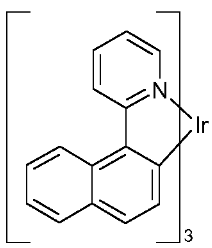

Formel (170)

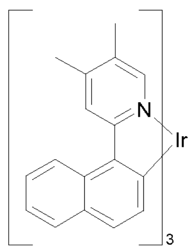

Formel (171)

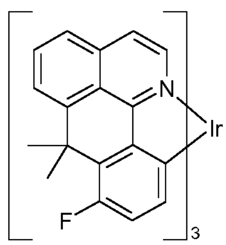

Formel (172)

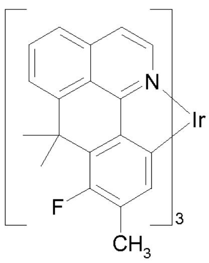
Formel (173)

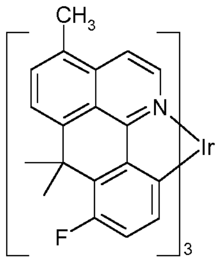
Formel (174)

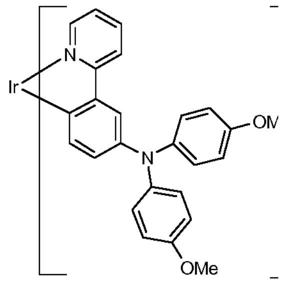
Formel (175)

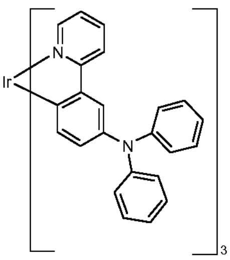
Formel (176)

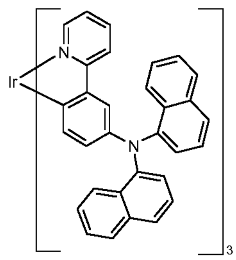
Formel (177)

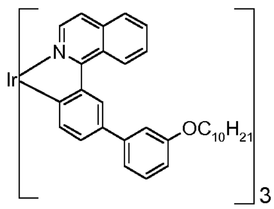
Formel (178)

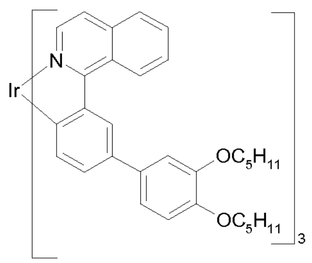
Formel (179)

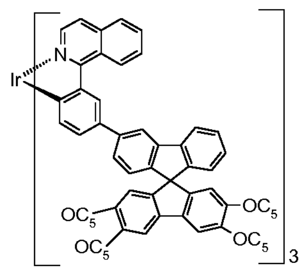
Formel (180)

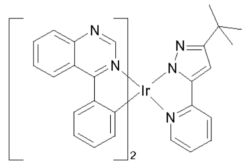
Formel (181)

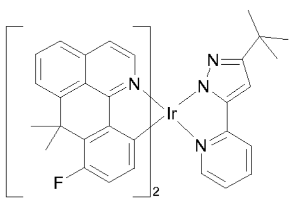
Formel (182)

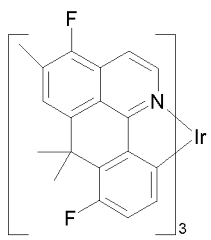
Formel (183)

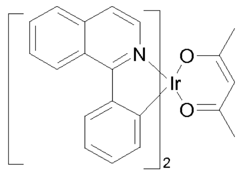
Formel (184)

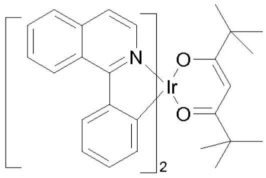

Formel (185)

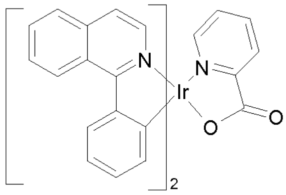

Formel (186)

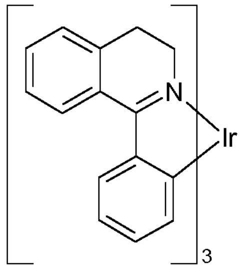

Formel (187)

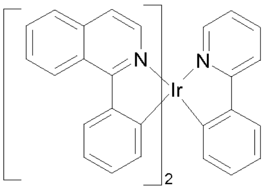

Formel (188)

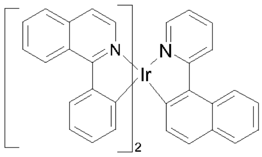

Formel (189)

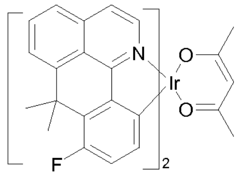

Formel (190)

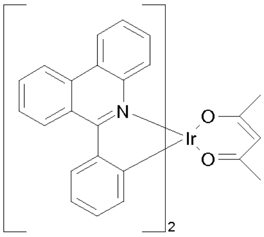

Formel (191)

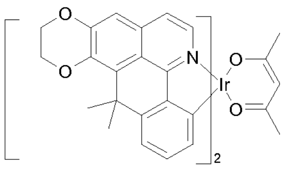

Formel (192)

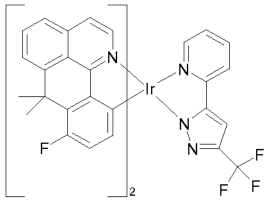

Formel (193)

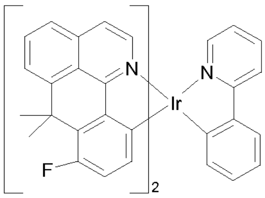

Formel (194)

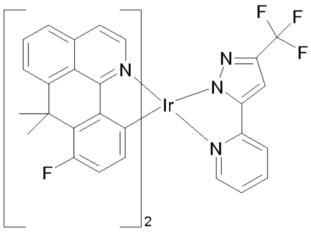

Formel (195)

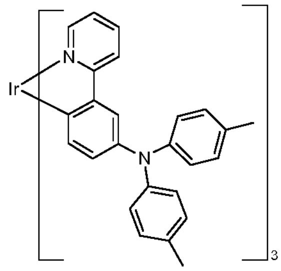

Formel (196)

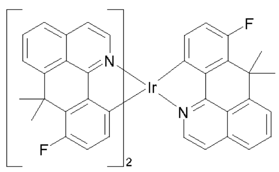
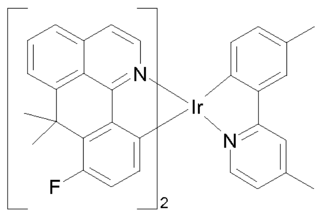
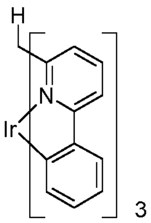

Formel (197) Formel (198) Formel (199)

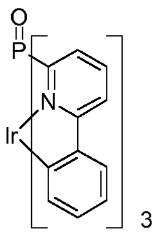
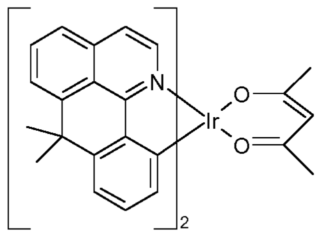
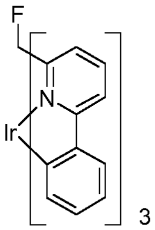

Formel (200) Formel (201) Formel (202)

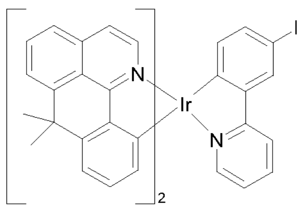
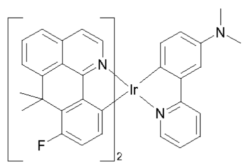
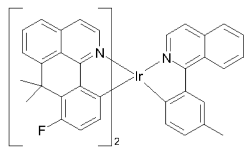

Formel (203) Formel (204) Formel (205)

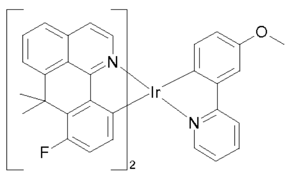
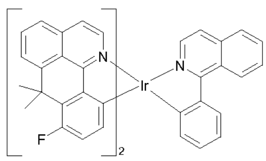
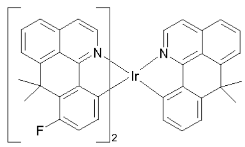

Formel (206) Formel (207) Formel (208)

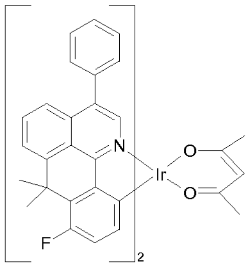

Formel (209)

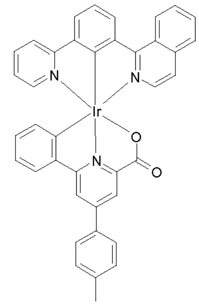

Formel (210)

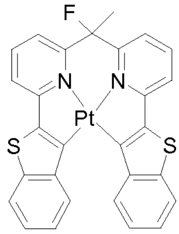

Formel (211)

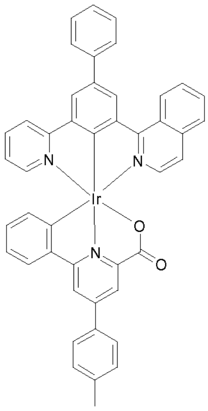

Formel (212)

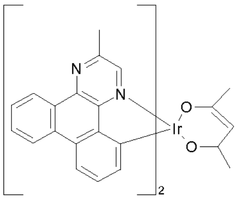

Formel (213)

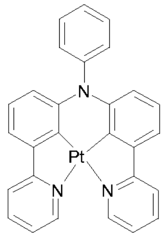

Formel (214)

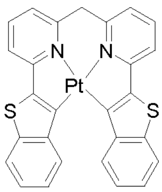

Formel (215)

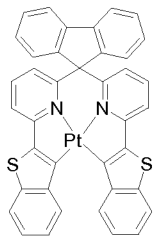

Formel (216)

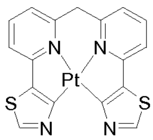

Formel (217)

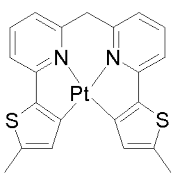

Formel (218)

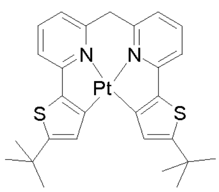

Formel (219)

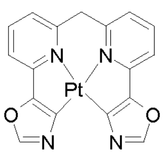

Formel (220)

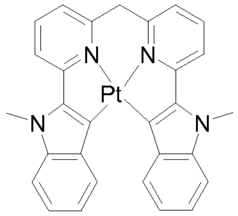

Formel (221)

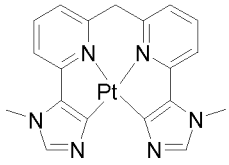

Formel (222)

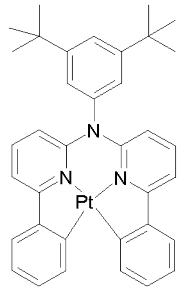

Formel (223)

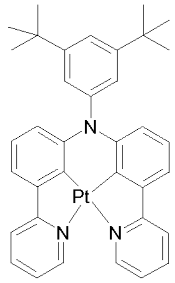

Formel (224)

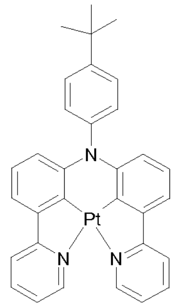

Formel (225)

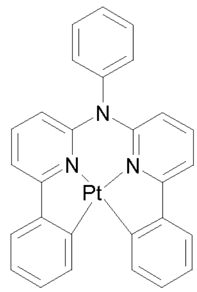

Formel (226)

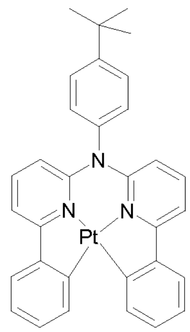

Formel (227)

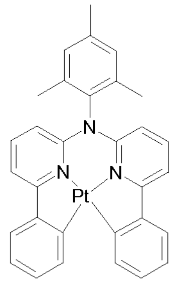

Formel (228)

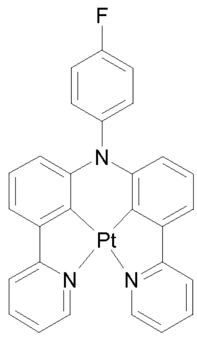

Formel (229)

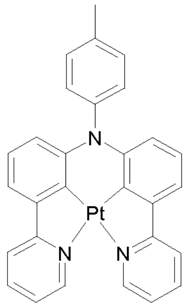

Formel (230)

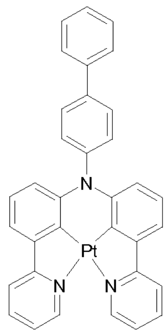

Formel (231)

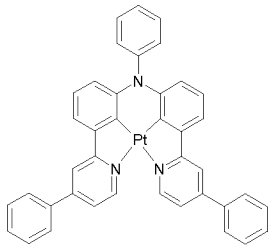

Formel (232)

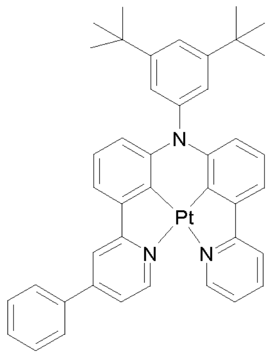

Formel (233)

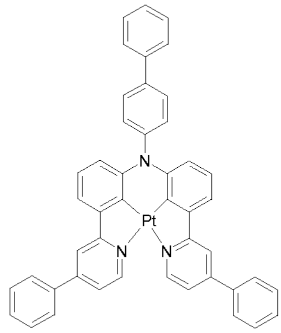

Formel (234)

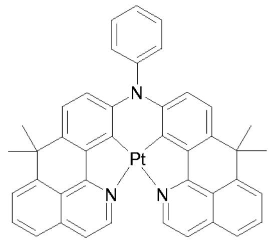

Formel (235)

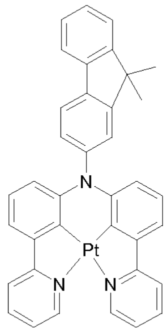

Formel (236)

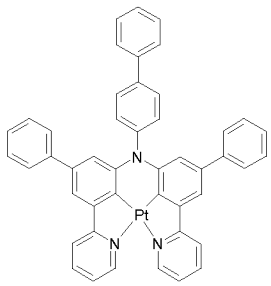

Formel (237)

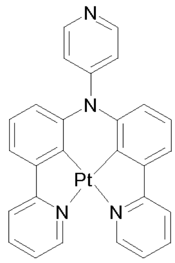

Formel (238)

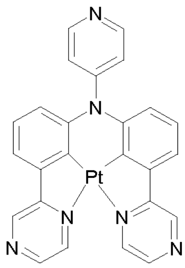

Formel (239)

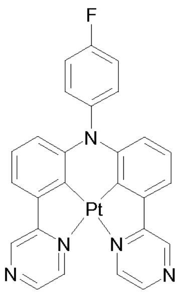

Formel (240)

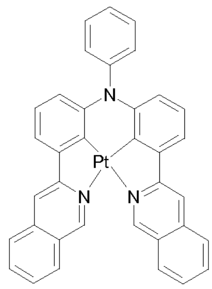

Formel (241)

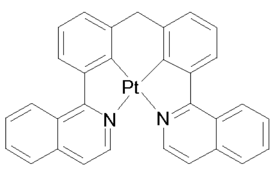

Formel (242)

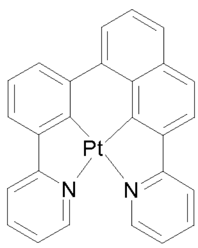

Formel (243)

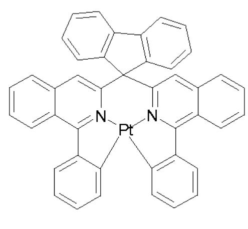

Formel (244)

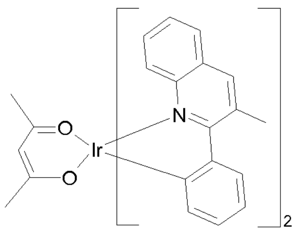

Formel (245)

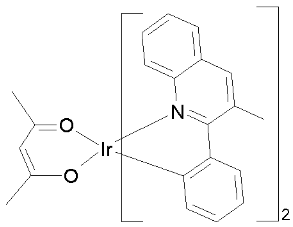

Formel (246)

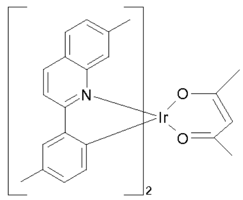

Formel (247)

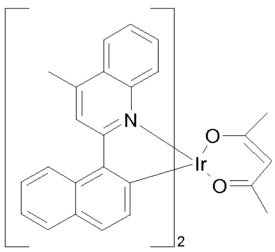

Formel (248)

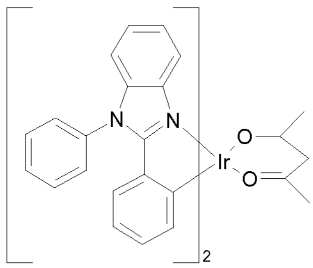

Formel (249)

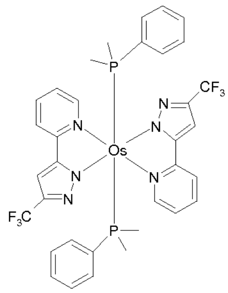

Formel (250)

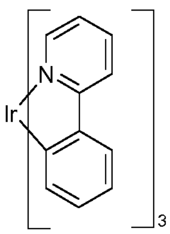

Formel (251)

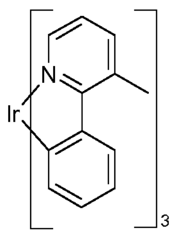

Formel (252)

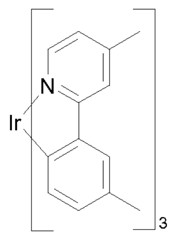

Formel (253)

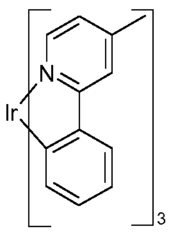

Formel (254)

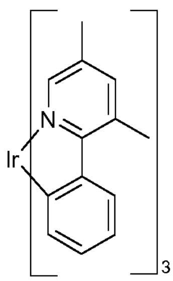

Formel (255)

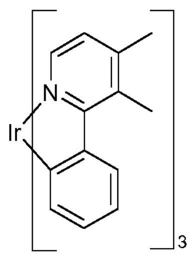

Formel (256)

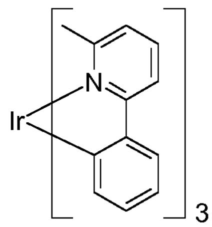

Formel (257)

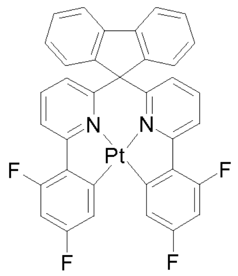

Formel (258)

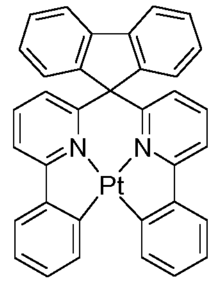

Formel (259)

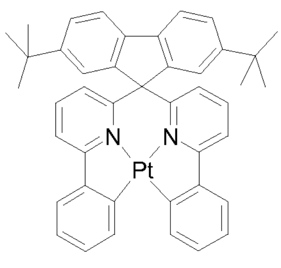

Formel (260)

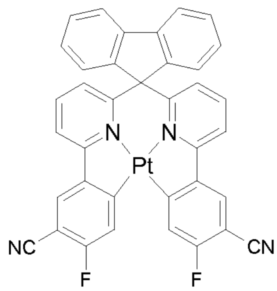

Formel (261)

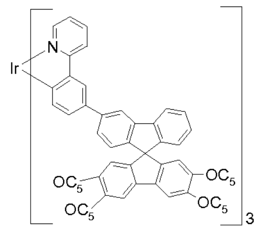

Formel (262)

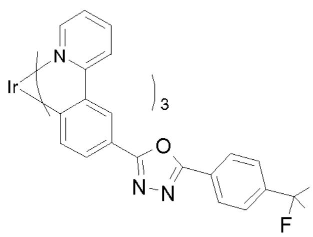

Formel (263)

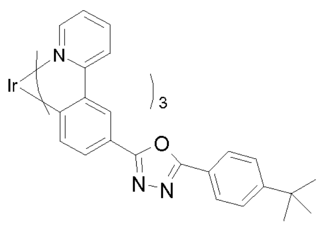

Formel (264)

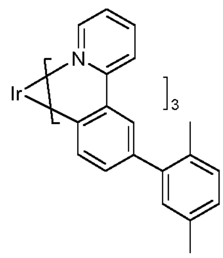

Formel (265)

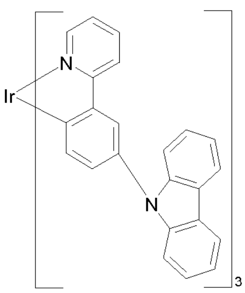

Formel (266)

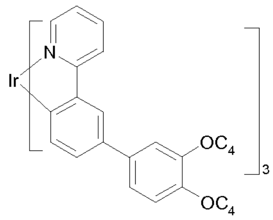

Formel (267)

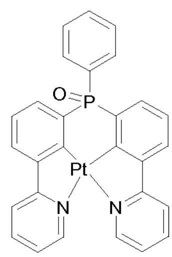

Formel (268)

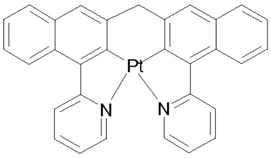

Formel (269)

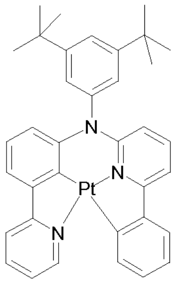

Formel (270)

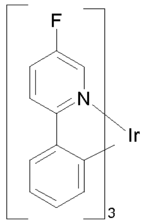

Formel (271)

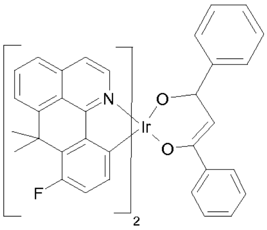

Formel (272)

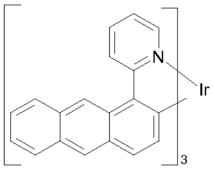

Formel (273)

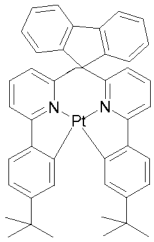

Formel (274)

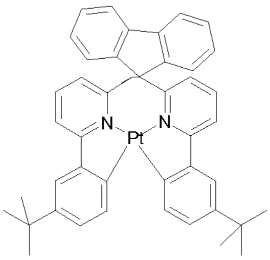

Formel (275)

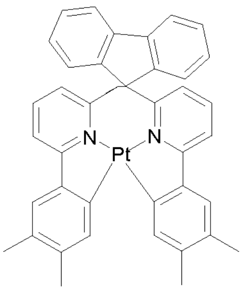

Formel (276)

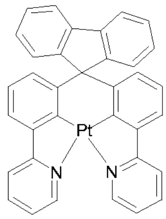

Formel (277)

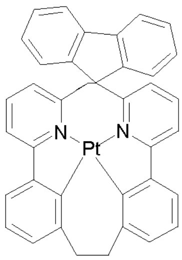

Formel (278)

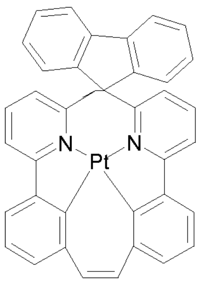

Formel (279)

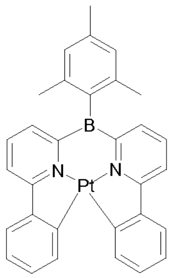

Formel (280)

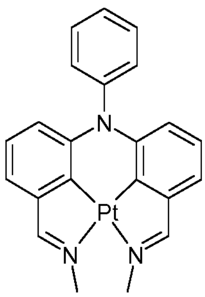

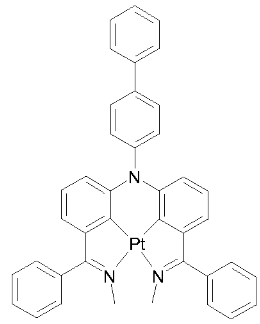

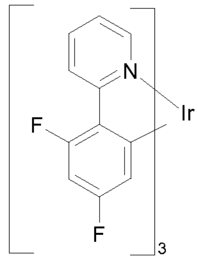

Formel (281) Formel (282) Formel (283)

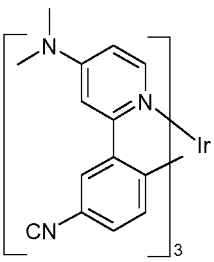

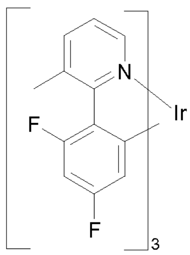

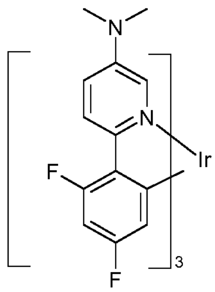

Formel (284) Formel (285) Formel (286)

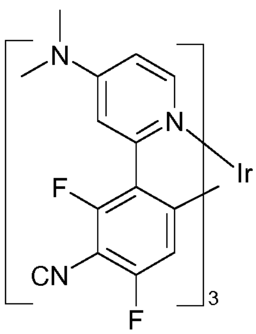

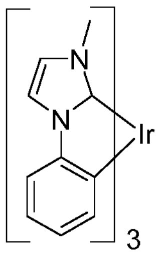

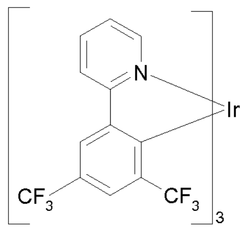

Formel (287) Formel (288) Formel (289)

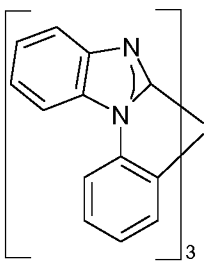

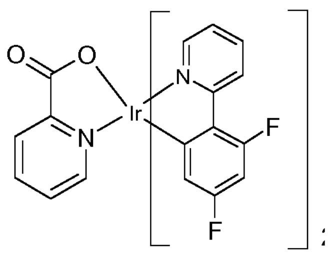

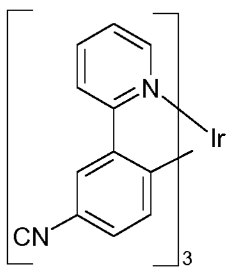

Formel (290) Formel (291) Formel (292)

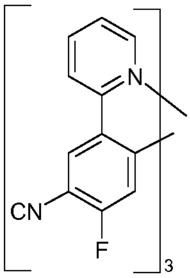

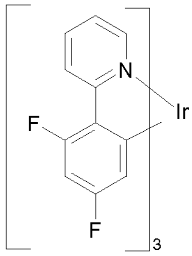

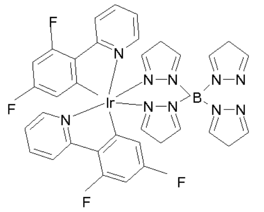

Formel (293) Formel (294) Formel (295)

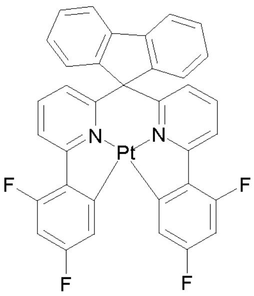

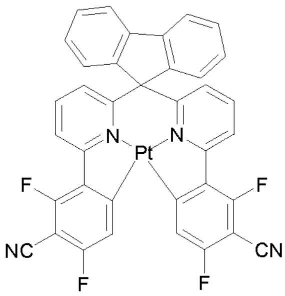

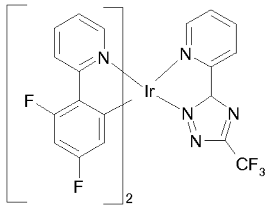

Formel (296) Formel (297) Formel (298)

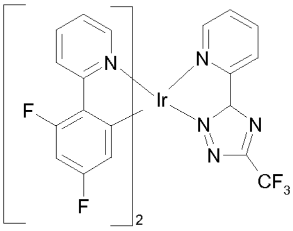

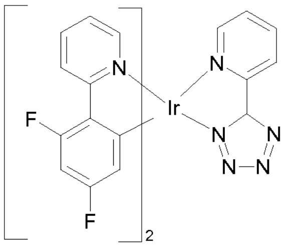

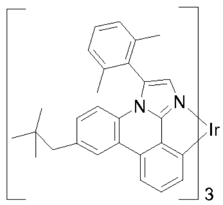

Formel (299) Formel (300) Formel (301)

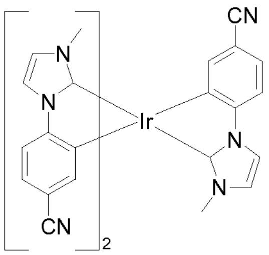

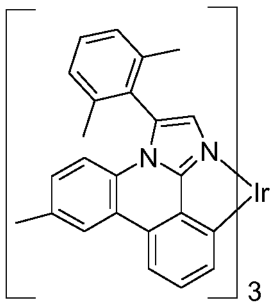

Formel (302) Formel (303)

In einer weiteren besonders bevorzugten Ausführungsform der Erfindung ist die erfindungsgemäße organische elektronische Vorrichtung eine, worin die Verbindung gemäß Formel (9) als Elektronentransportmaterial in einer Elektronentransportschicht (ETL) oder Elektroneninjektionsschicht (EIL) oder Lochblockierschicht (HBL) oder als Exzitonenblockiermaterial in einer Exzitonenblockierschicht (ExBL) eingesetzt wird. Dabei kann die organische elektronische Vorrichtung eine ETL oder EIL oder HBL oder ExBL enthalten, oder sie kann mehrere ETL oder EIL oder HBL oder ExBL enthalten, wobei mindestens eine Schicht von ETL oder EIL oder HBL oder ExBL mindestens eine Verbindung gemäß Formel (9) enthält.

In einer besonders bevorzugten Ausführungsform der vorliegenden Erfindung werden die Verbindungen der Formel (9) wie oben beschrieben als Matrixmaterialien eingesetzt.

In einer weiteren bevorzugten Ausführungsform der Erfindung enthält die erfindungsgemäße organische elektronische Vorrichtung in einer Elektronentransportschicht oder Elektroneninjektionsschicht mindestens eine Verbindung der Formel (9). Besonderes bevorzugt enthält die Elektronentransportschicht oder Elektroneninjektionsschicht noch mindestens ein weiteres Elektronentransportmaterial. Die für diese Verwendung bevorzugten Gruppen $Ar^1$ und/oder $Ar^2$ der Verbindung der Formel (9) sind oben ausführlich aufgeführt.

In einer bevorzugten Ausführungsform handelt es sich bei dem weiteren Elektronentransportmaterial um eine organische Alkalimetallverbindung.

Der Gesamtanteil der Verbindung gemäß Formel (9) in der Mischung mit dem weiteren Elektronentransportmaterial beträgt zwischen 20.0 und 99.0 mol%, bevorzugt zwischen 30.0 und 90.0 mol%, besonders bevorzugt zwischen 30.0 und 70.0 mol%. Entsprechend beträgt der Anteil des weiteren Elektronentransportmaterials zwischen 1.0 und 80.0 mol%, bevorzugt zwischen 10.0 und 70.0 mol%, besonders bevorzugt zwischen 30.0 und 70.0 mol%.

Unter einer organischen Alkalimetallverbindung im Sinne dieser Erfindung soll eine Verbindung verstanden werden, welche mindestens ein Alkalimetall, also Lithium, Natrium, Kalium, Rubidium oder Caesium, enthält und welche weiterhin mindestens einen organischen Liganden enthält.

Geeignete organische Alkalimetallverbindungen sind beispielsweise die in WO 2007/050301, WO 2007/050334 und EP 1144543 offenbarten Verbindungen.

Bevorzugte organische Alkalimetallverbindungen sind die Verbindungen der folgenden Formel (304),

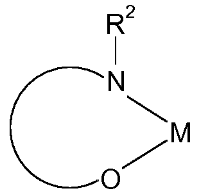

Formel (304)

wobei $R^2$ dieselbe Bedeutung hat, wie oben beschrieben, die gebogene Linie zwei oder drei Atome und Bindungen darstellt, welche erforderlich sind, um mit M einen 5- oder 6-Ring zu ergänzen, wobei diese Atome auch durch einen oder mehrere Reste $R^2$ (wie oben beschrieben) substituiert sein können, und M ein Alkalimetall, ausgewählt aus Lithium, Natrium, Kalium, Rubidium oder Caesium, darstellt.

Dabei ist es möglich, dass der Komplex gemäß Formel (304) in monomerer Form vorliegt, wie oben abgebildet, oder dass er in Form von Aggregaten vorliegt, beispielsweise aus zwei Alkalimetallionen und zwei Liganden, vier Alkalimetallionen und vier Liganden, sechs Alkalimetallionen und sechs Liganden oder in Form anderer Aggregate.

Bevorzugte Verbindungen der Formel (304) sind die Verbindungen der folgenden Formeln (305) und (306),

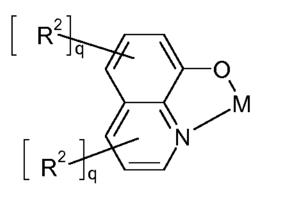

Formel (305)

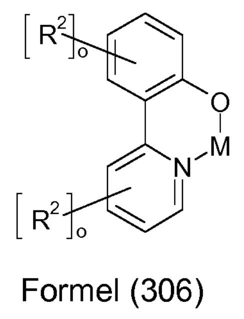

Formel (306)

wobei die verwendeten Symbole die oben genannten Bedeutungen haben und weiterhin gilt:

q ist bei jedem Auftreten gleich oder verschieden 0, 1, 2 oder 3;

o ist bei jedem Auftreten gleich oder verschieden 0, 1, 2, 3 oder 4.

Weitere bevorzugte organische Alkalimetallverbindungen sind die Verbindungen gemäß der folgenden Formel (306),

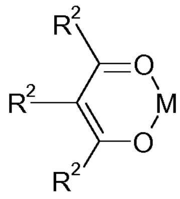

Formel (306)

wobei die verwendeten Symbole dieselbe Bedeutung haben, wie oben beschrieben.

Bevorzugt ist das Alkalimetall gewählt aus Lithium, Natrium und Kalium, besonders bevorzugt Lithium und Natrium, ganz besonders bevorzugt Lithium.

Besonders bevorzugt ist eine Verbindung der Formel (304), insbesondere mit M = Lithium. Ganz besonders bevorzugt sind weiterhin die Indizes q = 0. Ganz besonders bevorzugt handelt es sich also um unsubstituiertes Lithiumchinolinat.

Beispiele für geeignete organische Alkalimetallverbindungen sind die in der folgenden Tabelle aufgeführten Strukturen mit den Formeln (307) bis (351).

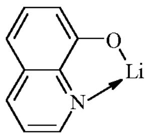

Formel (307)

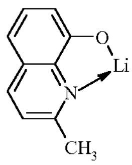

Formel (308)

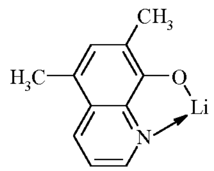

Formel (309)

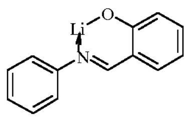

Formel (310)

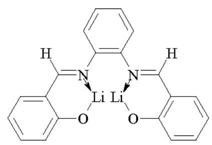

Formel (311)

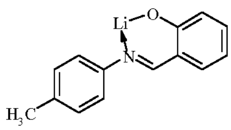

Formel (312)

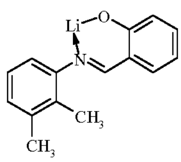

Formel (313)

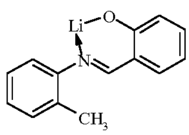

Formel (314)

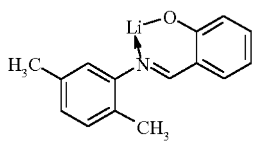

Formel (315)

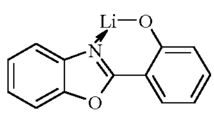

Formel (316)

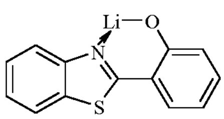

Formel (317)

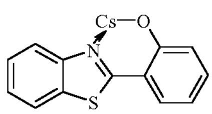

Formel (318)

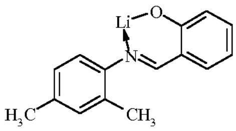

Formel (319)

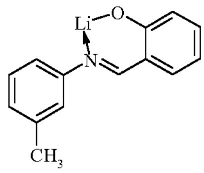

Formel (320)

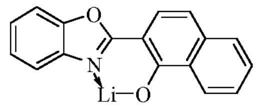

Formel (321)

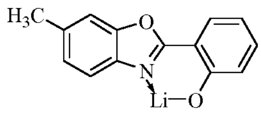

Formel (322)

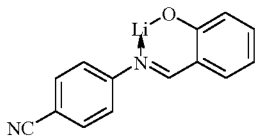

Formel (323)

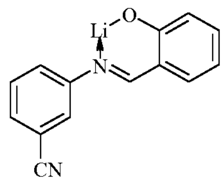

Formel (324)

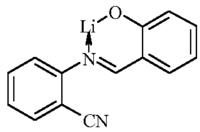

Formel (325)

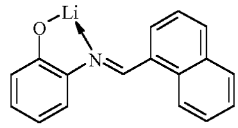

Formel (326)

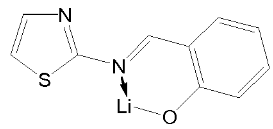

Formel (327)

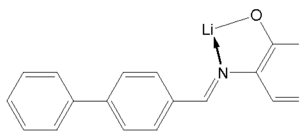

Formel (328)

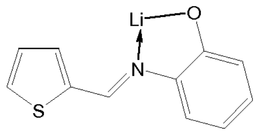

Formel (329)

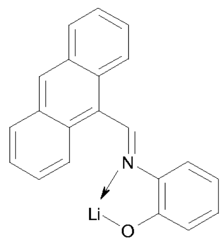

Formel (330)

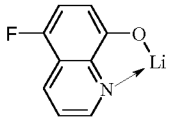

Formel (331)

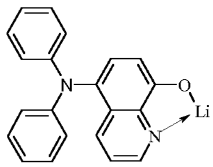

Formel (332)

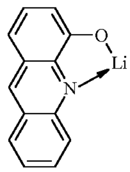

Formel (333)

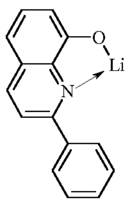

Formel (334)

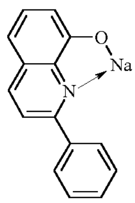

Formel (335)

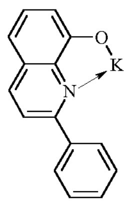

Formel (336)

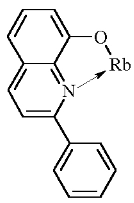
Formel (337)

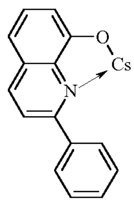
Formel (338)

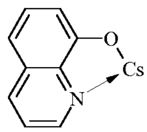
Formel (339)

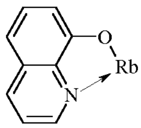
Formel (340)

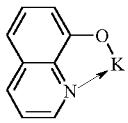
Formel (341)

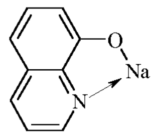
Formel (342)

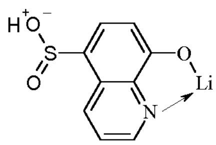
Formel (343)

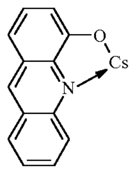
Formel (344)

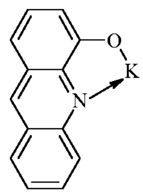
Formel (345)

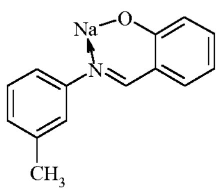
Formel (346)

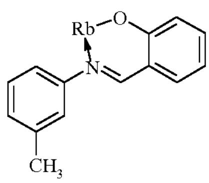
Formel (347)

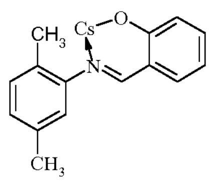
Formel (348)

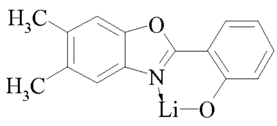
Formel (349)

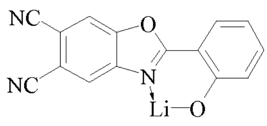
Formel (350)

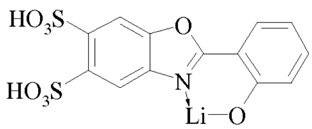
Formel (351)

Eine weitere besonders bevorzugten Ausführungsform der vorliegenden Erfindung ist eine organische elektronische Vorrichtung, worin die Verbindung gemäß Formel (9) als Lochtransportmaterial in einer Lochtransportschicht (HTL) oder Lochinjektionsschicht (EIL) oder Elektronblockierschicht (EBL) eingesetzt wird. Dabei kann die organische elektronische Vorrichtung eine HTL oder HIL oder EBL enthalten, oder sie kann mehrere HTL oder HIL oder EBL enthalten, wobei mindestens eine Schicht von HTL oder HIL oder EBL mindestens eine Verbindung gemäß Formel (9) enthält.

Die Verbindungen gemäß Formel (9), die für HTL oder HIL oder EBL geeignet sind, sind vorzugsweise solche bei denen wenigstens ein Vertreter der $Ar^1$ ein heteroaromatisches Ringsystem darstellt.

In einer besonders bevorzugten Ausführungsform der vorliegenden Erfindung wird eine Verbindung gemäß Formel (9) in HTL oder HIL oder EBL eingesetzt, wobei X und Y gleich $CR^1$ oder N und m eine ganze Zahl von 1 bis 6 ist, mit der Maßgabe, dass mindestens ein Vertreter der X oder Y gleich N ist.

Neben der wenigstens einen Schicht kann die erfindungsgemäße organische elektronische Vorrichtung noch

weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Charge-Generation Layers (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Außerdem können Interlayers vorhanden sein, welche die Ladungsbalance im Device steuern. Weiterhin können die Schichten, insbesondere die Ladungstransportschichten, auch dotiert sein. Die Dotierung der Schichten kann für einen verbesserten Ladungstransport vorteilhaft sein. Es sei aber darauf hingewiesen, dass nicht notwendigerweise jede dieser Schichten vorhanden sein muss und die Wahl der Schichten immer von den verwendeten Verbindungen abhängt.

In einer weiteren bevorzugten Ausführungsform der Erfindung enthält die organische elektronische Vorrichtung mehrere emittierende Schichten, wobei mindestens eine emittierende Schicht mindestens eine Verbindung gemäß Formel (9) und mindestens einer phosphoreszierenden Emitterverbindung enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Insbesondere bevorzugt sind Dreischichtsysteme, also Systeme mit drei emittierenden Schichten, wobei mindestens eine dieser Schichten mindestens eine Verbindung gemäß Formel (9) und mindestens eine phosphoreszierende Emitterverbindung enthält und wobei die drei Schichten blaue, grüne und orange oder rote Emission zeigen (für den prinzipiellen Aufbau siehe z. B.
WO 2005/011013). Auch die Verwendung von mehr als drei emittierenden Schichten kann bevorzugt sein. Ebenso eignen sich für weiße Emission Emitter, welche breitbandige Emissionsbanden aufweisen und dadurch weiße Emission zeigen.

In einer weiteren Ausführungsform der vorliegenden Erfindung ist es bevorzugt, dass die wenigstens eine Schicht eine oder mehrere weitere Verbindungen enthält, die aus den folgenden ausgewählt werden: Lochinjektionsverbindungen, Lochtransportverbindungen, Lochblockierverbindungen, Elektronentransportverbindungen, Elektroneninjektionsverbindungen, Elektronenblockierverbindungen, Excitonenblockierverbindungen. Insbesondere enthält die wenigstens eine Schicht eine Lochtransportverbindung, bevorzugt ausgewählt aus Triarylaminen, Carbazolderivaten, Azacarbazolen und bipolaren Matrixmaterialien.

Die vorliegende Erfindung betrifft auch eine Zusammensetzung enthaltend mindestens eine Verbindung gemäß Formel (9) wie in Anspruch 13 definiert, und mindestens eine phosphoreszierende Emitterverbindung, wie vorstehend definiert.

Die erfindungsgemäße Zusammensetzung aus der Verbindung gemäß Formel (9) und der phosphoreszierenden Emitterverbindung enthält vorzugsweise zwischen 99 und 50 Vol.-%, vorzugsweise zwischen 98 und 50 Vol.-%, besonders bevorzugt zwischen 97 und 60 Vol.-%, insbesondere zwischen 95 und 85 Vol.-% der Verbindung gemäß Formel (9) bezogen auf die Gesamtmischung aus Emitterverbindung und Matrixmaterial. Entsprechend enthält die Mischung zwischen 1 und 50 Vol.-%, vorzugsweise zwischen 2 und 50 Vol.-%, besonders bevorzugt zwischen 3 und 40 Vol.-%, insbesondere zwischen 5 und 15 Vol.-% der phosphoreszierenden Emitterverbindung bezogen auf die Gesamtmischung aus Emitter und Matrixmaterial.

Bevorzugt ist weiterhin auch die Verwendung mehrerer Matrixmaterialien in der erfindungsgemäßen Zusammensetzung, wobei ein Matrixmaterial ausgewählt ist aus Verbindungen der Formel (9). Die Verbindungen gemäß Formel (9) haben überwiegend elektronentransportierende Eigenschaften durch die elektronenarmen Stickstoffheterocyclen $Ar^1$ oder $Ar^2$. Wenn eine Mischung aus zwei oder mehr Matrixmaterialien verwendet wird, ist daher eine weitere Komponente der Mischung bevorzugt eine lochtransportierende Verbindung. Bevorzugte lochleitende Matrixmaterialien sind Triarylamine, Carbazolderivate, z. B. CBP (N,N-Biscarbazolylbiphenyl) oder die in WO 2005/039246, US 2005/0069729, JP 2004/288381, EP 1205527 oder WO 08/086851 offenbarten Carbazolderivate, Azacarbazole, z. B. gemäß EP 1617710, EP 1617711, EP 1731584, JP 2005/347160, bipolare Matrixmaterialien, z. B. gemäß WO 2007/137725, und 9,9-Diarylfluorenderivate, z. B. gemäß der Anmeldung DE 102008017591.9.

Die Zusammensetzung aus mehreren Matrixmaterialien kann auch mehr als zwei Matrixmaterialien enthalten. Es ist weiterhin auch möglich, das Matrixmaterial gemäß Formel (9) als Mischung mit einem weiteren elektronentransportierenden Matrixmaterial zu verwenden. Bevorzugte weitere elektronentransportierende Matrixmaterialien sind Ketone, z. B. gemäß WO 2004/093207, Phosphinoxide, Sulfoxide und Sulfone, z. B. gemäß WO 2005/003253, Oligophenylene, bipolare Matrixmaterialien, z. B. gemäß WO 07/137725, Silane, z. B. gemäß WO 05/111172, 9,9-Diarylfluorenderivate (z. B. gemäß der nicht offen gelegten Anmeldung DE 102008017591.9), Azaborole oder Boronester (z. B. gemäß WO 06/117052).

Weiterhin bevorzugt ist eine organische elektronische Vorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner $10^{-5}$ mbar, bevorzugt kleiner $10^{-6}$ mbar aufgedampft. Es sei jedoch angemerkt, dass der Druck auch noch geringer sein kann, beispielsweise kleiner $10^{-7}$ mbar.

Bevorzugt ist ebenfalls eine organische elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen. Dabei können nicht nur Lösungen aus einzelnen Materialien aufgebracht werden, sondern auch Lösungen, die mehrere Verbindungen enthalten, beispielsweise Matrixmaterialien und Dotanden.

Die organische elektronische Vorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine Verbindung gemäß Formel (9) und einen phosphoreszierenden Dotanden aus Lösung aufzubringen und darauf eine Lochblockierschicht und/oder eine Elektronentransportschicht im Vakuum aufzudampfen. Ebenso kann die emittierende Schicht enthaltend eine Verbindung gemäß Formel (9) und einen phosphoreszierenden Dotanden im Vakuum aufgedampft werden und eine oder mehrere andere Schichten können aus Lösung aufgebracht werden.

Diese Verfahren sind dem Fachmann generell bekannt und können von ihm ohne Probleme auf organische elektronische Vorrichtungen enthaltend Verbindungen gemäß Formel (9) bzw. die oben aufgeführten bevorzugten Ausführungsformen angewandt werden.

Nochmals ein weiterer Gegenstand der vorliegenden Erfindung ist die Verwendung von Verbindungen gemäß Formel (9) als Matrixmaterial für phosphoreszierende Emitterverbindungen in einer organischen elektronischen Vorrichtung, insbesondere einer organischen Elektrolumineszenzvorrichtung.

Die erfindungsgemäße organische elektronische Vorrichtung enthält vorzugsweise eine Kathode und eine Anode, die vorzugsweise an zwei gegenüberliegenden Seiten der wenigstens einen Schicht der Vorrichtung angebracht sind. Die Elektroden (Kathode, Anode) werden im Sinne dieser Erfindung so gewählt, dass ihr Potential möglichst gut mit dem Potential der angrenzenden organischen Schicht übereinstimmt, um eine möglichst effiziente Elektronen- bzw. Lochinjektion zu gewährleisten.

Als Kathode sind Metallkomplexe, Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide in Frage (z.B. LiF, $Li_2O$, $BaF_2$, MgO, NaF, etc.). Die Schichtdicke dieser Schicht beträgt bevorzugt zwischen 1 und 10 nm, mehr bevorzugt 2 bis 8 nm.

Als Anode sind Materialien mit hoher Austrittsarbeit bevorzugt. Bevorzugt weist die Anode ein Potential größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektronen (z. B. Al/Ni/$NiO_x$, Al/$PtO_x$) bevorzugt sein. Für einige Anwendungen muss mindestens eine der Elektroden transparent sein, um entweder die Bestrahlung des organischen Materials (O-SC) oder die Auskopplung von Licht (OLED/PLED, O-LASER) zu ermöglichen. Ein bevorzugter Aufbau verwendet eine transparente Anode. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

Die Vorrichtung wird in an sich bekannter Weise je nach Anwendung entsprechend strukturiert, kontaktiert und schließlich hermetisch versiegelt, da sich die Lebensdauer derartiger Vorrichtungen bei Anwesenheit von Wasser und/oder Luft drastisch verkürzt.

Die erfindungsgemäßen organischen elektronischen Vorrichtungen weisen folgende überraschende Vorteile gegenüber dem Stand der Technik auf:

1. Die erfindungsgemäßen organischen elektronischen Vorrichtungen weisen eine sehr hohe Effizienz auf.

2. Die erfindungsgemäßen organischen elektronischen Vorrichtungen weisen gleichzeitig eine verringerte Betriebsspannung auf.

Die vorliegende Erfindung betrifft auch eine organische Verbindung der Formel (9) mit der Maßgabe, dass wenigstens ein Vertreter der Y gleich N und wenigstens ein Vertreter der X gleich $CR^1$ ist, wobei Y, X und $R^1$ ansonsten die gleichen Bedeutungen wie in Anspruch 1 definiert aufweisen. Alle oben genannten bevorzugten Definitionen gelten auch für diese erfindungsgemäße organische Verbindung.

Die erfindungsgemäßen Verbindungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der oben ausgeführten erfindungsgemäßen Verbindungen in einer organisch elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen. Dabei gelten die oben ausgeführten Bevorzugungen ebenso für die elektronischen Vorrichtungen.

Die elektronische Vorrichtung ist bevorzugt ausgewählt aus der Gruppe bestehend aus organischen Elektrolumineszenzvorrichtungen (organischen Leuchtdioden, OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen lichtemittierenden elektrochemischen Transistoren, organischen Solarzellen (O-SCs), farbstoffsensibilisierten organischen Solarzellen (ODSSCs), organischen optischen Detektoren, organischen Photorezeptoren, organischen Feld-Quench-Devices (O-FQDs), organischen lichtemittierenden elektrochemischen Zellen (OLECs), organischen Laserdioden (O-Laser) und "organic plasmon emitting devices" (D. M. Koller et al., Nature Photonics 2008, 1-4), bevorzugt aber organischen Elektrolumineszenzvorrichtungen (OLEDs), besonders bevorzugt phosphoreszierenden OLEDs, OLEC, O-LET und organischen lichtemittierenden elektrochemischen Transistor.

Die hierin beschriebenen Verbindungen eignen sich, wie oben beschrieben, zur Verwendung in O-FETs, was weiter unten im Detail gezeigt wird. Typischerweise werden in O-FETs konjugierte Polymere oder Oligomere, wie zum beispiele Thiophen-enthaltenden Polymer P3HT, oder Makromoleküle, beispielweise Phthalocyanine und deren Derivative eingesetzt. Thiophene-Polymere zeigen nach wie vor Probleme bei der Prozessierung aus Lösungen sowie bei der Aufreinigung. Die Phthalocyanin-basierten Materialen lassen sich nur durch Verdampfen auftragen. Die Verwendung der hierin offenbarten kleinen Molekülverbindungen ist vorteilhaft gegenüber diesen, da sie leichter prozessierbar aus Lösung sind. Weiterhin ist ihre Synthese vergleichsweise einfach und sie können in höherer Reinheit gewonnen werden, was einen positiven Einfluss auf die Performance der elektronischen Vorrichtungen hat.

Weiterhin bevorzugt ist die erfindungsgemäße organische elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist aber auch möglich, dass der Anfangsdruck noch geringer ist, beispielsweise kleiner $10^{-7}$ mbar.

Bevorzugt ist die erfindungsgemäße organische elektronische Vorrichtung dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische elektronische Vorrichtung, dadurch gekennzeichnet, dass eine oder mehrere Schichten aus Lösung, wie z. B. durch Spincoating, oder mit einem beliebigen Druckverfahren, wie z. B. LITI (Light Induced Thermal Imaging, Thermotransferdruck), Ink-Jet Druck (Tintenstrahldruck), Siebdruck, Flexodruck, Offsetdruck oder Nozzle Printing, hergestellt werden. Hierfür sind lösliche Verbindungen nötig, welche beispielsweise durch geeignete Substitution erhalten werden. Diese Verfahren eignen sich auch für Oligomere, Dendrimere und Polymere. Diese Verfahren eignen sich insbesondere auch für die erfindungsgemäßen Verbindungen, da diese allgemein eine sehr gute Löslichkeit in organischen Lösemitteln aufweisen.

Weiterhin sind Hybridverfahren möglich, bei denen beispielsweise eine oder mehrere Schichten aus Lösung aufgebracht werden und eine oder mehrere weitere Schichten aufgedampft werden. So kann beispielsweise in einer organischen elektronischen Vorrichtung die eine Schicht auf Lösung aufgebracht werden und die andere Schicht aufgedampft werden.

Diese Verfahren sind dem Fachmann generell bekannt und können von ihm ohne erfinderisches Zutun auf die oben beschriebenen organischen elektronischen Vorrichtungen enthaltend die erfindungsgemäßen Verbindungen angewandt werden.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen oder der Verbindungen der Formel (9) nach Anspruch 1 erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden.

Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Formulierung, insbesondere eine Lösung, Dispersion oder Miniemulsion, enthaltend mindestens eine erfindungsgemäße Verbindung und mindestens ein Lösungsmittel, insbesondere ein organisches Lösungsmittel. Wie solche Lösungen hergestellt werden können, ist dem Fachmann bekannt und beispielsweise in der WO 02/072714, der WO 2003/019694 und der darin zitierten Literatur beschrieben.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird.

Geeignete phosphoreszierende Dotanden sind oben im Zusammenhang mit den erfindungsgemäßen organischen elektronischen Vorrichtungen aufgeführt und sind auch für die erfindungsgemäßen Mischungen bevorzugt.

Bevorzugte erfindungsgemäße organische Verbindungen der Formel (9) oder in der erfindungsgemäßen organischen elektronischen Vorrichtung eingesetzte Verbindungen der Formel (9) sind die folgenden:

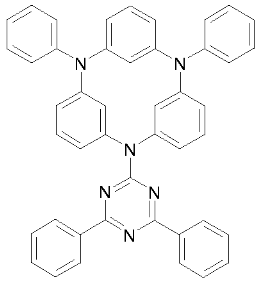

Formel (352)

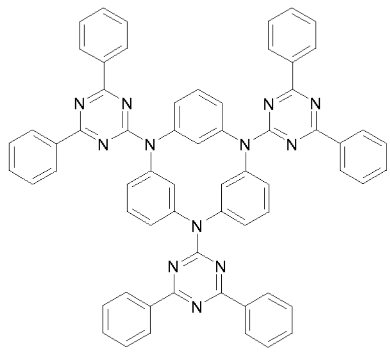

Formel (353)

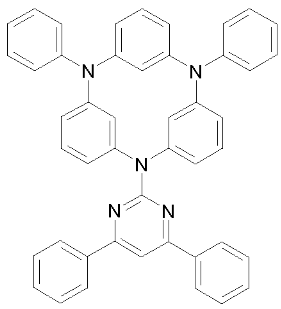

Formel (354)

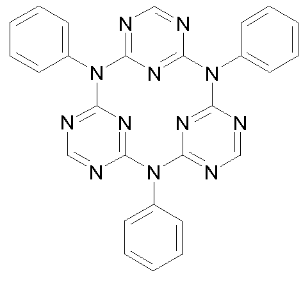

Formel (355)

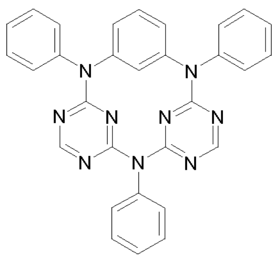

Formel (356)

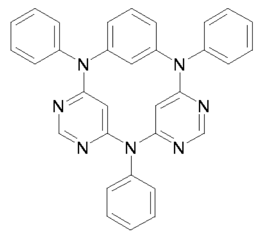

Formel (357)

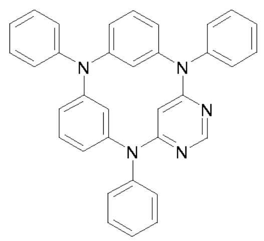

Formel (358)

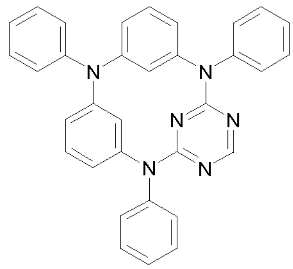

Formel (359)

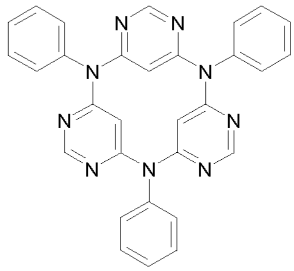

Formel (360)

Die erfindungsgemäßen Vorrichtungen enthaltend die oben angegebenen Verbindungen können in elektrolumineszierenden Verrichtungen eingesetzt werden. Sie eignen sich daher auch zum Einsatz in den Bereichen der therapeutischen und kosmetischen Phototherapie.

Eine mögliche Verwendung ist daher die Verwendung der Vorrichtungen enthaltend die Verbindungen nach Formel (1) oder (9) zur Behandlung, Prophylaxe und Diagnose von Erkrankungen oder. die Verwendung, der erfindungsgemäßen Verbindungen und Vorrichtungen enthaltend die Verbindungen zur Behandlung und Prophylaxe kosmetischer Umstände.

Phototherapie oder Lichttherapie findet in vielen medizinischen und/oder kosmetischen Bereichen Anwendung. Die erfindungsgemäßen Vorrichtungen und Verbindungen nach Formel (9) können daher zur Therapie und/oder Prophylaxe und/oder Diagnose von allen Erkrankungen und/oder in kosmetischen Anwendungen eingesetzt werden, für die der Fachmann die Anwendung von Phototherapie in Betracht zieht. Der Begriff Phototherapie beinhaltet dabei neben der Bestrahlung auch die photodynamische Therapie (PDT) sowie das Desinfizieren und Sterilisieren im Allgemeinen. Behandelt werden können mittels Phototherapie oder Lichttherapie nicht nur Menschen oder Tiere, sondern auch jegliche andere Art lebender oder unbelebter Materie. Hierzu gehören, bspw., Pilze, Bakterien, Mikroben, Viren, Eukaryonten, Prokaryonten, Nahrungsmittel, Getränke, Wasser und Trinkwasser.

Der Begriff Phototherapie beinhaltet auch jede Art der Kombination von Lichttherapie und anderen Therapiearten, wie bspw. die Behandlung mit Wirkstoffen. Viele Lichttherapien habe zum Ziel äußere Partien eines Objektes zu bestrahlen oder zu behandeln, so wie die Haut von Menschen und Tieren, Wunden, Schleimhäute, Auge, Haare, Nägel, das Nagelbett, Zahnfleisch und die Zunge. Die Behandlung oder Bestrahlung kann daneben auch innerhalb eines Objektes durchgeführt werden, um bspw. innere Organe (Herz, Lunge etc.) oder Blutgefäße oder die Brust zu behandeln.

Die therapeutischen und/oder kosmetischen Anwendungsgebiete sind bevorzugt ausgewählt aus der Gruppe der Hauterkrankungen und Haut-assoziierten Erkrankungen oder Veränderungen bzw. Umstände wie bspw. Psoriasis, Hautalterung, Hautfaltenbildung, Hautverjüngung, vergrößerte Hautporen, Cellulite, ölige/fettige Haut, Follikulitis, aktinische Keratose, Precancerose aktinische Keratose, Haut Läsionen, sonnengeschädigte und sonnengestresste Haut, Krähenfüße, Haut Ulkus, Akne, Akne rosacea, Narben durch Akne, Akne Bakterien, Photomodulierung fettiger/öliger Talgdrüsen sowie deren umgebende Gewebe, Ikterus, Neugeborenenikterus, Vitiligo, Hautkrebs, Hauttumore, Crigler Naijar, Dermatitis, atopische Dermatitis, diabetische Hautgeschwüre sowie Desensibilisierung der Haut.

Besonders bevorzugt sind die Behandlung und/oder Prophylaxe von Psoriasis, Akne, Cellulite, Hautfaltenbildung, Hautalterung, Ikterus und Vitiligo.

Weitere Anwendungsgebiete für die Zusammensetzungen und/oder Vorrichtungen enthaltend die erfindungsgemäßen Zusammensetzungen sind ausgewählt aus der Gruppe der Entzündungserkrankungen, rheumatoide Arthritis, Schmerztherapie, Behandlung von Wunden, neurologische Erkrankungen und Umstände, Ödeme, Paget's Erkrankung, primäre und metastasierende Tumoren, Bindegewebserkrankungen bzw. -veränderungen, Veränderungen des Kollagens, Fibroblasten und von Fibroblasten stammende Zellspiegel in Geweben von Säugetieren, Bestrahlung der Retina, neovasculare und hypertrophe Erkrankungen, allergische Reaktionen, Bestrahlung der Atemwege, Schwitzen, okulare neovaskulare Erkrankungen, virale Infektionen besonders Infektionen durch Herpes Simplex oder HPV (Humane Papillomviren) zur Behandlung von Warzen und Genitalwarzen.

Besonders bevorzugt sind die Behandlung und/oder Prophylaxe von rheumatoider Arthritis, viraler Infektionen, und Schmerzen.

Weitere Anwendungsgebiete für die Verbindungen und/oder Vorrichtungen enthaltend die Verbindungen sind ausgewählt aus der Winterdepression, Schlafkrankheit, Bestrahlung zur Verbesserung der Stimmung, Linderung von Schmerzen besonders Muskelschmerzen durch bspw. Verspannungen oder Gelenkschmerzen, Beseitigung der Steifheit von Gelenken und das Aufhellen der Zähne (Bleaching).

Weitere Anwendungsgebiete für die Verbindungen und/oder Vorrichtungen enthaltend die Verbindungen sind ausgewählt aus der Gruppe der Desinfektionen. Mit den Verbindungen und/oder mit den erfindungsgemäßen Vorrichtungen können jegliche Art von Objekten (unbelebte Materie) oder Subjekten (lebende Materie wie bspw. Mensch und Tier) zum Zweck der Desinfektion behandelt werden. Hierzu zählt, zum Beispiel, die Desinfektion von Wunden, die Reduktion von Bakterien, das Desinfizieren chirurgischer Instrumente oder anderer Gegenstände, das Desinfizieren von Nahrungs- und Lebensmitteln, von Flüssigkeiten, insbesondere Wasser, Trinkwasser und andere Getränke, das Desinfizieren von Schleimhäuten und Zahnfleisch und Zähnen. Unter Desinfektion wird hierbei die Reduktion lebender mikrobiologischer Verursacher unerwünschter Effekte, wie Bakterien und Keime, verstanden.

Zu dem Zweck der oben genannten Phototherapie, emittieren die erfindungsgemäßen Vorrichtungen bevorzugt Licht der Wellenlänge zwischen 250 and 1250 nm, besonders bevorzugt zwischen 300 and 1000 nm und insbesondere bevorzugt zwischen 400 and 850 nm.

Es können die Verbindungen der Formel (9) in einer organischen lichtemittierenden Diode (OLED) oder einer organischen lichtemittierenden elektrochemischen Zelle (OLEC) zum Zwecke der Phototherpie eingesetzt werden. Sowohl die OLED als auch die OLEC können dabei einen planaren oder Fiber- bzw. Faser-artigen Aufbau mit beliebigem Querschnitt (z.B. rund, oval, polygonal, quadratisch) mit einem ein- oder mehrschichtigen Aufbau aufweisen. Diese OLECs und/oder OLEDs können in andere Vorrichtungen eingebaut werden, die weitere mechanische, adhäsive und/oder elektronische Bausteine (z.B. Batterie und/oder Steuerungseinheit zur Einstellung der Bestrahlungszeiten, -intensitäten und -wellenlängen) enthalten. Diese Vorrichtungen enthaltend die erfindungsgemäßen OLECs und/order OLEDs sind vorzugsweise ausgewählt aus der Gruppe enthaltend Pflaster, Pads, Tapes, Bandagen, Manschetten, Decken, Hauben, Schlafsäcken,Textilien und Stents.

Die Verwendung von den genannten Vorrichtungen zu dem genannten therapeutischen und/oder kosmetischen Zweck ist besonders vorteilhaft gegenüber dem Stand der Technik, da mit Hilfe der erfindungsgemäßen Vorrichtungen unter Verwendung der OLEDs und/oder OLECs homogene Bestrahlungen geringerer Bestrahlungsintensitäten an nahezu jedem Ort und zu jeder Tageszeit möglich sind. Die Bestrahlungen können stationär, ambulant und/oder selbst, d.h., ohne Anleitung durch medizinisches oder kosmetisches Fachpersonal durchgeführt werden. So können, bspw., Pflaster unter der Kleidung getragen werden, so dass eine Bestrahlung auch während der Arbeitszeit, in der Freizeit oder während des Schlafes möglich ist. Auf aufwendige stationäre/ambulante Behandlungen kann in vielen Fällen verzichtet bzw. deren Häufigkeit reduziert werden. Die erfindungsgemäßen Vorrichtungen können zum Widergebrauch gedacht sein oder Wegwerfartikel darstellen, die nach ein-, zwei oder dreimaligem Gebrauch entsorgt werden können.

Weitere Vorteile gegenüber dem Stand der Technik sind bspw. eine geringere Wärmeentwicklung und emotionale Aspekte. So werden Neugeborene, die aufgrund einer Gelbsucht (Ikterus) therapiert werden müssen typischerweise mit verbundenen Augen in einem Brutkasten, ohne körperlichen Kontakt zur den Eltern bestrahlt, was eine emotionale Stresssituation für Eltern und Neugeborene darstellt. Mit Hilfe einer Decke enthaltend die erfindungsgemäßen OLEDs und/oder OLECs kann der emotionale Stress signifikant vermindert werden. Zudem ist eine bessere Temperierung des Kindes durch eine verringerte Wärmeproduktion der erfindungsgemäßen Vorrichtungen gegenüber herkömmlicher Bestrahlungsgeräte möglich.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn, dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die Erfindung wird durch die nachfolgenden Beispiele näher erläutert, ohne sie dadurch einschränken zu wollen.

## Beispiele

### Beispiele 1

### Quantenchemische Berechnungen zu M1 - M8, V1 und TEG1

Zuerst werden die folgenden organischen Verbindungen durch die Quantum Chemie Simulation untersucht, wobei die Verbindungen M5 und M6 die erfindungsgemäßen Verbindungen sind, M1, M2, M7 und M8 nicht erfindungsgemäß sind und M3 und M4 in den erfindungsgemäßen organischeen Elektrolumineszenzvorrichtungen verwendet werden können, V1 eine Vergleich-Matrix-Material, und TEG1 ein grüner Triplett-Emitter ist.

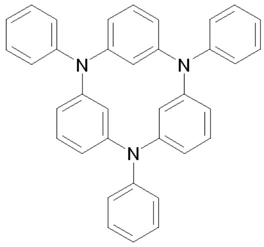

M1

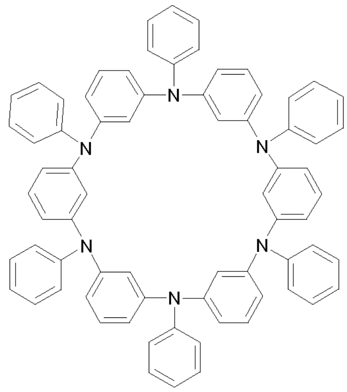

M2

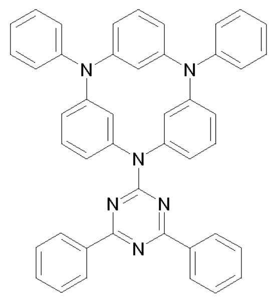

M3

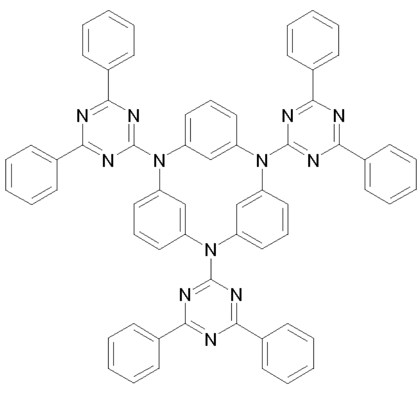

M4

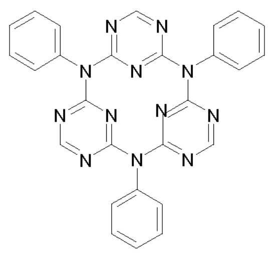

M5

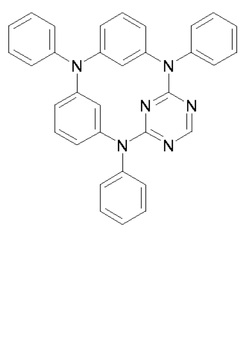

M6

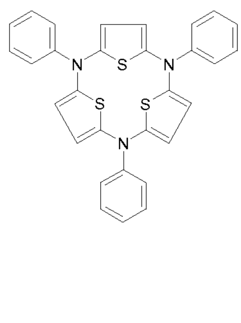

M7

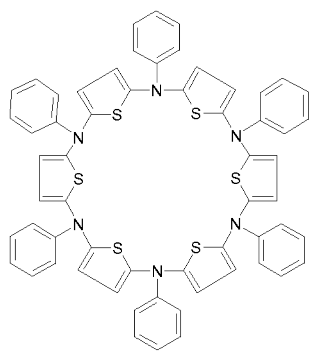

M8

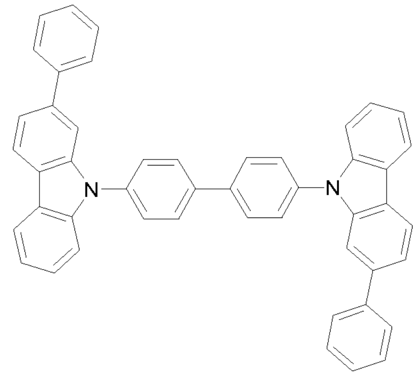

V1

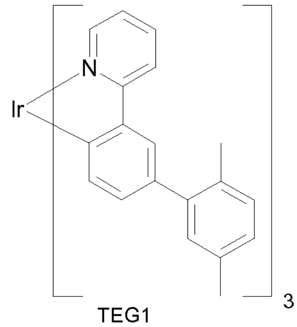

TEG1

HOMO- und LUMO-Lagen sowie das Triplett/Singlett Niveau organischer Verbindungen lassen sich mittels quanten-chemischer Rechnungen bestimmen. Hierzu wird die Software "Gaussian03W" (Gaussian Inc.) verwendet. Zur Berechnung organischer Substanzen ohne Metalle wird zuerst eine Geometrieoptimierung mit eine Semi-empirische Methode "Ground State/Semi-empirical/ Default Spin/AM1" (Charge 0/Spin Singlet) durchgeführt. Im Anschluss hierzu erfolgt auf Grundlage der optimierten Geometrie eine Energierechnung. Hierbei wird die Methode "TD-SCF/DFT/ Default Spin/B3PW91" (TD-SCF/DFT - time dependent-self consisiting field/density functional theory) mit dem Basissatz "6-31 G(d)" verwendet (Charge 0/Spin Singlet). Für metall-organische Verbindungen wird die Geometrie über die Methode "Ground State/Hartree-Fock/Default Spin/LanL2MB" (Charge 0/Spin Singlet) optimiert. Die Energierechnung erfolgt analog zu den organischen Substanzen wie oben beschrieben mit dem Unterschied, dass für das Metallatom der Basissatz "LanL2DZ" (pseudo=LanL2) und für die Liganden der Basissatz "6-31 G(d)" verwendet wird. Die wichtigsten Ergebnisse aus solchen Rechnungen sind die HOMO / LUMO-Niveaus und die Energien angeregter Triplett-und Singulett-Zustände. Die ersten angeregten Zustände (Singulett und Triplett) sind hierbei am wichtigsten. Sie werden als T1 (erster angeregter Triplett-Zustand) und S1 (erster angeregter Singulett-Zustand) bezeichnet. Aus der Energierechnung erhält man das HOMO HEh bzw. LUMO LEh in Hartree-Einheiten. Daraus werden die HOMO- und LUMO-Werte in Elektronenvolt wie folgt bestimmt, wobei sich diese Beziehungen aus der Kalibrierung anhand von Cyclovoltammetriemessungen (CV) ergeben:

$$\text{HOMO(eV)} = ((\text{HEh}*27.212)-0.9899)/1.1206$$

$$\text{LUMO(eV)} = ((\text{LEh}*27.212)-2.0041)/1.385$$

Diese Werte sind im Sinne dieser Anmeldung als energetische Lage des HOMO-Niveaus bzw. des LUMO-Niveaus der Materialien anzusehen. Als Beispiel erhält man für die Verbindung M1 (siehe auch Tabelle 1) aus der Rechnung ein HOMO von -0.17968 Hartrees und ein LUMO von - 0.02961Hartrees, was einem kalibrierten HOMO von -5.25 eV, einem kalibrierten LUMO von -2.03 eV entspricht.

Die berechneten Energieniveaus sind in Tabelle 1 zusammengefasst. Die T1-Niveaus von M1 bis M6 und M8 sind höher als die von TEG1, was darauf hindeutet, dass alle diese Materialien geeignete Matrixmaterialien für TEG1 sind.

**Tabelle 1**: Zusammenfassung der Energieniveaus von M1 bis M8, V1 und TEG1

| | TD-DFT | | | |
|---|---|---|---|---|
| | Homo Corr. [eV] | Lumo Corr. [eV] | T1 [eV] | S1 [eV] |
| M1 | -5.25 | -2.03 | 2.80 | 3.17 |
| M2 | -5.69 | -2.16 | 3.22 | 3.75 |
| M3 | -5.36 | -2.68 | 2.74 | 2.92 |
| M4 | -5.90 | -2.75 | 2.80 | 3.04 |
| M5 | -6.55 | -2.88 | 3.02 | 3.41 |
| M6 | -5.36 | -2.18 | 2.94 | 3.45 |

(fortgesetzt)

| | TD-DFT | | | |
|---|---|---|---|---|
| | Homo Corr. [eV] | Lumo Corr. [eV] | T1 [eV] | S1 [eV] |
| M7 | -5.35 | -2.62 | 2.06 | 2.63 |
| M8 | -5.79 | -2.28 | 2.96 | 3.08 |
| V1 | -5.69 | -2.39 | 2.85 | 3.28 |
| TEG1 | -5.33 | -2.41 | 2.68 | 2.91 |

Wie man auch aus Tabelle 1 entnehmen kann, haben die Verbindungen M6 und M7 ein sehr hoch liegendes HOMO und sind folglich sehr gut als HTM geeignet. Man kann diese Verbindungen als HTM in HTL in OLED oder in organischen Solarzellen oder in einem p-Transport -Kanal in "organic field effect transistor" einsetzen.

**Beispiele 2**

**Synthese von M1 (nicht erfindungsgemäß)**

Die Verbindung M1 wird nach dem folgenden Schema synthetisiert.

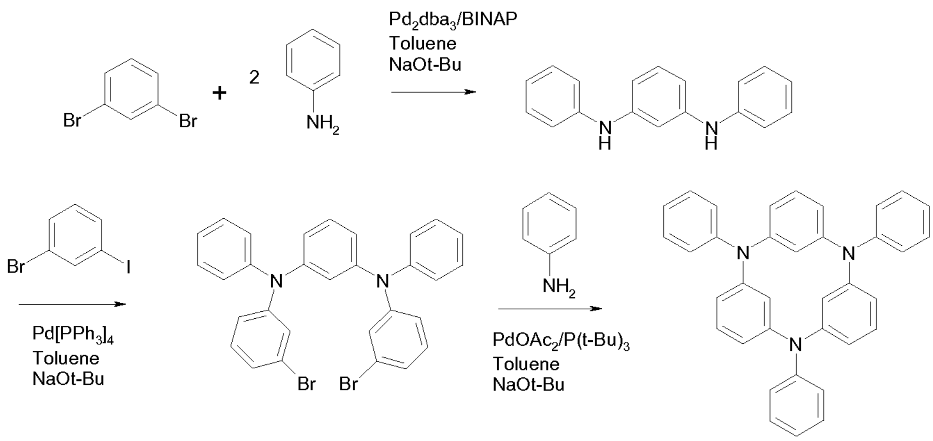


**a) Synthese von N,N-Diphenyl-1,3-diaminobenzol**

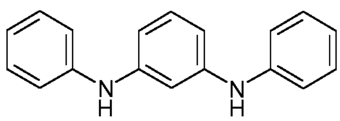

23.1 g (100 mmol) 1.3 Dibrombenzol [108-36-1], 23.3 g (250 mmol) Anilin [62-53-3] und 28.8 g (300 mmol) Natert.-Butylat [865-45-5] werden in 300 ml Toluol gelöst. Die Reaktionslösung wird sorgfällig entgast, auf 80°C erwärmt und mit 183.2 mg (0.2 mmol) $Pd_2(dba)_3$ und 373.6 mg (0.6 mmol) rac-BINAP als Katalysator versetzt. Der Reaktionsfortschritt wird mittels DC kontrolliert. Die Lösung wird auf Raumtemperatur abgekühlt mit 150 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen. Der Rückstand wird aus Ethanol umkristallisiert. Man erhält 19.3 g (74 mmol) (74%) eines weißen Feststoffes in einer von Reinheit 99.2%.

**b) Synthese von N,N-Diphenyl-N,N-bis-3-Bromphenyldiaminobenzol**

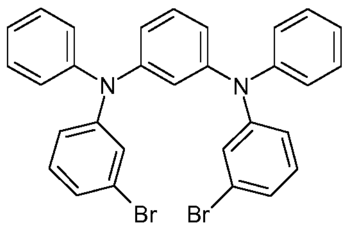

18 g (69 mmol) 1.3 Diphenyldiaminobenzol, 14 g (139.4 mmol) Bromiodbenzol [591-81-4] und 20.2 g (210 mmol) Na-tert.-Butylat [865-45-5] werden in 100 ml Toluol gelöst. Die Reaktionslösung wird sorgfällig entgast, auf 80°C erwärmt und mit 31 mg (0.14 mmol) $Pd(OAc)_2$ und 127.8 mg $P(t\text{-}Bu)_3$ als (0.42 mmol) Katalysator versetzt. Der Reaktionsfortschritt wird mittels DC kontrolliert. Die Lösung wird auf Raumtemperatur abgekühlt mit 50 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen. Der Rückstand wird aus Ethanol umkristallisiert. Man erhält 26.8 g (47 mmol) (68%) eines weißen Feststoffes in einer von Reinheit 99.3%.

c) Synthese von Cyclophan M1

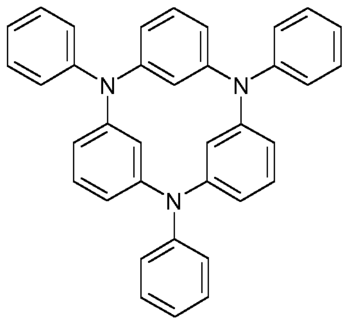

25 g (44 mmol) Dibromid, 4.08 g (44 mmol) Anilin [62-53-3] und 12.7 g (132 mmol) Na-tert.-Butylat [865-45-5] werden in 500 ml Toluol gelöst. Die Reaktionslösung wird sorgfällig entgast, auf 80°C erwärmt und mit 16 mg (0.07 mmol) $Pd(OAc)_2$ und 64 mg $P(t\text{-}Bu)_3$ als (0.21 mmol) als Katalysator versetzt. Der Reaktionsfortschritt wird mittels DC kontrolliert. Die Lösung wird auf Raumtemperatur abgekühlt mit 100 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen. Der Rückstand wird mehrfach aus Buthanol umkristallisiert. Man erhält 9.3 g (18.5 mmol) (42%) eines weißen Feststoffes in einer von Reinheit 99.9%.

## Beispiele 3

### Synthese von M3

Die Verbindung M3 wird nach dem folgenden Schema synthetisiert.

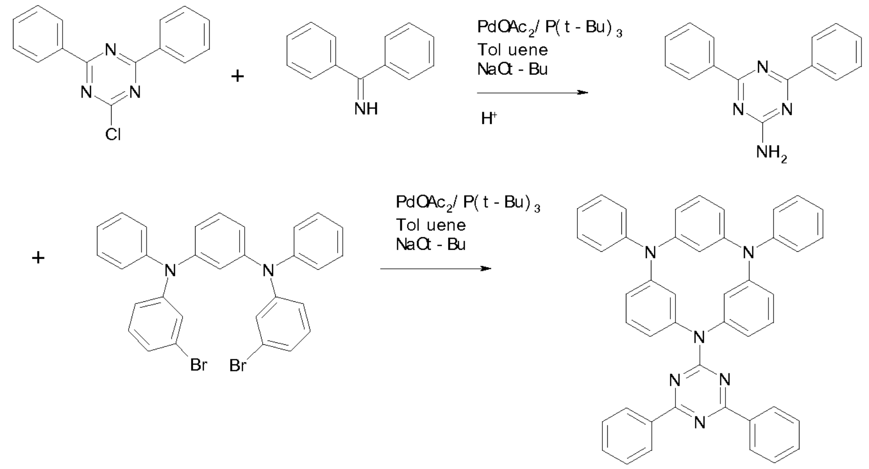


**a) Synthese von 2-Amino-4,6-Diphenyl-1,3,5 Triazin**

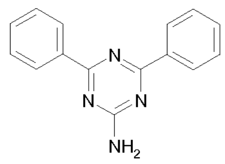

25 g (93.4 mmol) Diphenylchlortriazin [3842-55-5], 18.1 g (100 mmol) Benzophenonimid [1013-88-3] und 14.4 g (150 mmol) Na-tert.-Butylat [865-45-5] werden in 200 ml Toluol gelöst. Die Reaktionslösung wird sorgfällig entgast, auf 80°C erwärmt und 16 mg (0.07 mmol) $Pd(OAc)_2$ und 64 mg $P(t\text{-}Bu)_3$ als (0.21 mmol) als Katalysator versetzt Der Reaktionsfortschritt wird mittels DC kontrolliert. Die Lösung wird auf Raumtemperatur abgekühlt mit 100 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen. Der Rückstand in Essigester aufgenommen und mit 10 ml Trifluoressigsäure versetzt um das Imid zu hydrolisieren. Der Rückstand wird aus Toluol/Ethanol 1:1 umkristallisiert. Man erhält 18.8 g (75.6 mmol) (81%) eines weißen Feststoffes in einer von Reinheit 99.3%.

**b) Synthese von Cyclophan M3**

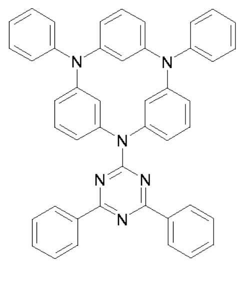

10 g (17.5 mmol) Dibromid, 4.3 g (17.5 mmol) Aminotriazin und 2.88 g (30 mmol) Na-tert.-Butylat [865-45-5] werden in 300 ml Toluol gelöst. Die Reaktionslösung wird sorgfältig entgast, auf 80°C erwärmt 20 mg (0.09 mmol) $Pd(OAc)_2$ und 82 mg $P(t\text{-}Bu)_3$ als (0.27 mmol) als Katalysator versetzt. Der Reaktionsfortschritt wird mittels DC kontrolliert. Die Lösung wird auf Raumtemperatur abgekühlt mit 100 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen und mehrfach aus Isopropanol umkristallisiert. Man erhält 4.4 g (6.65 mmol) (38%) eines weißen Feststoffes in einer von Reinheit 99.9%.

**Beispiele 4:**

**Synthese von M6**

Die Verbindung M6 wird nach dem folgenden Schema synthetisiert.

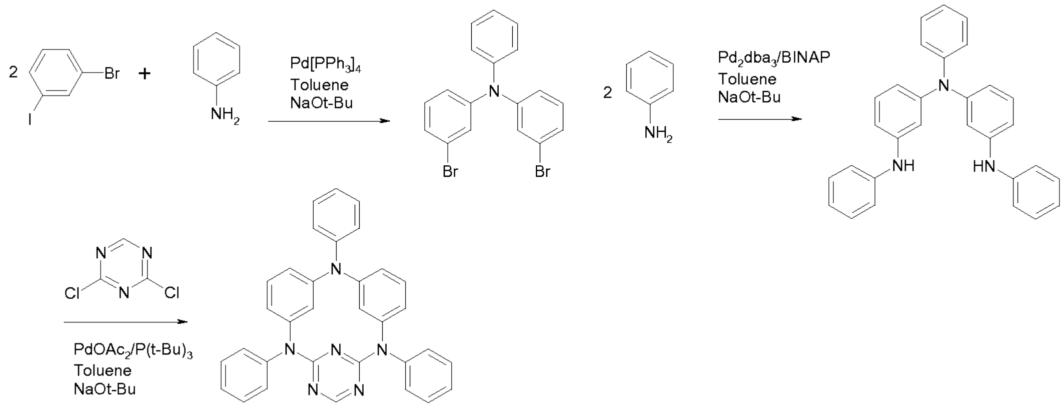


**a) Synthese von 3,3'-Dibromtriarylamin**

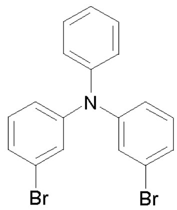

50 g (177 mmol) Bromiodbenzol [591-81-4], 8.2 g (88 mmol) Anilin [62-53-3] und 28.8 g (300 mmol) Na-tert.-Butylat [865-45-5] werden in 300 ml Toluol gelöst. Die Reaktionslösung wird sorgfällig entgast, auf 110°C erwärmt und mit 40 mg (0.18 mmol) $Pd(OAc)_2$ und 164 mg $P(t\text{-}Bu)_3$ als (0.54 mmol) als Katalysator versetzt. Der Reaktionsfortschritt wird mittels DC kontrolliert. Die Lösung wird auf Raumtemperatur abgekühlt mit 50 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen. Der Rückstand wird aus Hexan umkristallisiert. Man erhält 23.4 g (58 mmol) (66%) eines weißen Feststoffes in einer von Reinheit 99.7%.

**b) Synthese von -N,N-diphenyl-3,3'-Diaminotriarylamin**

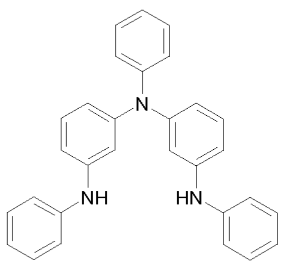

15 g (37.2 mmol) Dibromtriarylamin, 6.9 g (74.4 mmol) Anilin [62-53-3] und 9.6 g (100 mmol) Na-tert.-Butylat [865-45-5] werden in 200 ml Toluol gelöst. Die Reaktionslösung wird sorgfällig entgast, auf 100°C erwärmt und mit 92

mg (0.1 mmol) $Pd_2(dba)_3$ und 187 mg (0.3 mmol) rac-BINAP als Katalysator versetzt. Der Reaktionsfortschritt wird mittels DC kontrolliert. Die Lösung wird auf Raumtemperatur abgekühlt mit 50 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen. Der Rückstand wird aus Hexan umkristallisiert. Man erhält 8.2 g (19.3 mmol) (52%) eines weißen Feststoffes in einer von Reinheit 99.1%.

**c) Synthese von Cyclophan M6**

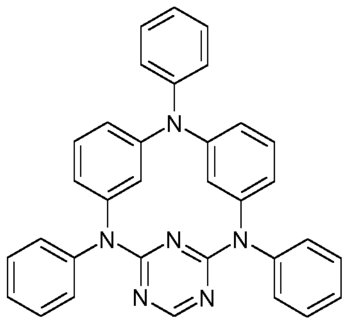

7 g (16.4 mmol) Diamin, 2.5 g (16.4 mmol) Dichlortriazin [2831-66-5] und 4.8 g (50 mmol) Na-tert.-Butylat [865-45-5] werden in 500 ml Toluol gelöst.

Die Reaktionslösung wird sorgfällig entgast, auf 80°C erwärmt und mit 40 mg (0.18 mmol) $Pd(OAc)_2$ und 164 mg (0.54 mmol) $P(t\text{-}Bu)_3$ als Katalysator versetzt. Der Reaktionsfortschritt wird mittels DC kontrolliert. Die Lösung wird auf Raumtemperatur abgekühlt mit 100 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen und mehrfach aus Ethanol umkristallisiert. Man erhält 3.1 g (6.2 mmol) (38%) eines weißen Feststoffes in einer von Reinheit 99.9%.

**Beispiele 5**

**Synthese von M7 (nicht erfindungsgemäß)**

Die Verbindung M7 wird nach dem folgenden Schema synthetisiert.

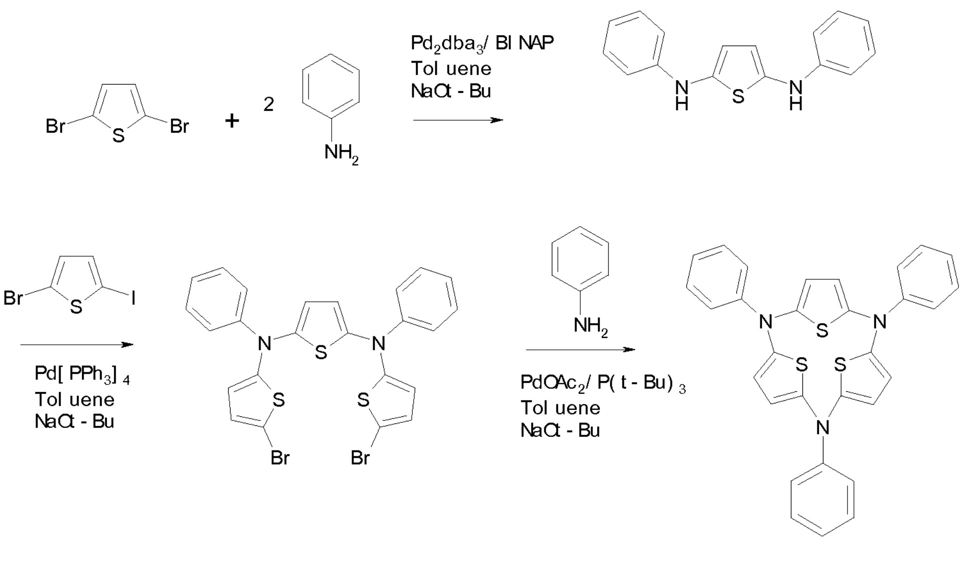

**a) Synthese von N,N-Diphenyl-1,3-diaminothiophen**

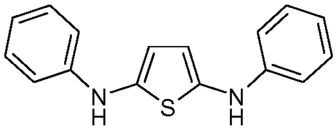

50 g (207 mmol) 1.3 Dibromthiophen [3141-27-4], 39.1 g (420 mmol) Anilin und 57.7 g (600 mmol) Na-tert.-Butylat [865-45-5] werden in 1000 ml Toluol gelöst. Die Reaktionslösung wird sorgfällig entgast, auf 90°C erwärmt und mit 276 mg (0.3 mmol) $Pd_2(dba)_3$ und 561 mg (0.9 mmol) rac-BINAP als Katalysator versetzt. Der Reaktionsfortschritt wird mittels DC kontrolliert. Die Lösung wird auf Raumtemperatur abgekühlt mit 50 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen. Der Rückstand wird aus 1-Propanol umkristallisiert. Man erhält 40.8 g (153 mmol) (74%) eines weißen Feststoffes in einer von Reinheit 99.8%.

**b) Synthese von N,N-Diphenyl-N,N-bis-3-Bromthiophendiaminothiophen**

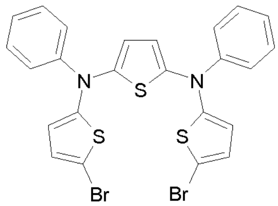

30 g (112.6 mmol) 1.3 Diphenyldiaminothiophen, 65.1 g (225 mmol) Bromiodthiophen [29054-81-2] und 28.8 g (300 mmol) Na-tert.-Butylat [865-45-5] werden in 500 ml Toluol gelöst. Die Reaktionslösung wird sorgfällig entgast, auf 90°C erwärmt und mit 80 mg (0.36 mmol) $Pd(OAc)_2$ und 328 mg (0.108 mmol) $P(t\text{-}Bu)_3$ als Katalysator versetzt. Der Reaktionsfortschritt wird mittels DC kontrolliert. Die Lösung wird auf Raumtemperatur abgekühlt mit 50 ml $H_2O$ versetzt und die Phasen getrennt. Die wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen. Der Rückstand wird aus Buthanol umkristallisiert. Man erhält 44.8 g (76.6 mmol) (68%) eines weißen Feststoffes in einer von Reinheit 99.4%.

**d) Synthese von Cyclophan M7**

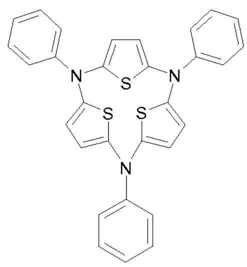

30 g (51.2 mmol) Dibromid, 4.8 g (51.2 mmol) Anilin [62-53-3] und 14.4 g (150 mmol) Na-tert.-Butylat [865-45-5] werden in 300 ml Toluol gelöst. Die Reaktionslösung wird sorgfällig entgast, auf 80°C erwärmt und mit 40 mg (0.18 mmol) $Pd(OAc)_2$ und 164 mg (0.54 mmol) $P(t\text{-}Bu)_3$ als Katalysator versetzt. Der Reaktionsfortschritt wird mittels DC kontrolliert. Die Lösung wird auf Raumtemperatur abgekühlt mit 100 ml $H_2O$ versetzt und die Phasen getrennt. Die

wässrige Phase wird dreimal mit Toluol extrahiert, anschließend die vereinigten organischen Phasen zweimal mit Wasser gewaschen, über Magnesiumsulfat getrocknet, filtriert und das Lösungsmittel im Vakuum abgezogen. Der Rückstand wird mehrfach aus Buthanol umkristallisiert. Man erhält 7.4 g (14.3 mmol) (28%) eines weißen Feststoffes in einer von Reinheit 99.9%.

**Beispiele 6**

**Verwendete Materialien in organischen elektronisch Vorrichtungen**

Die folgenden Materialien werden verwendet: V1 ist ein Referenz-Matrixmaterial gemäß dem Stand der Technik (WO 2008/086851). M1, M3 und M6 sind Matrixmaterialien, deren Synthesen in den Beispielen 2 bis 4 beschrieben sind. M6 und M7 können als HTM oder HIM verwendet werden. TEG1 ist ein phosphoreszierender Emitter, wobei TEG für Triplettemitter Grün steht. TEG1 wurde nach WO 2004/026886 synthetisiert. Die Synthese von V1 erfolgt nach WO 2008/086851.

Das Matrixmaterial TMM1 wird nach WO 2004/093207 synthetisiert, und wird im Folgenden als Co-Matrix verwendet.

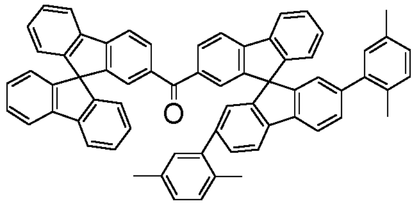

TMM1

Das Polymer HIL-012 (Merck KGaA) wird als Interlayer verwendet.

**Beispiel 7**

**Lösungen und Zusammensetzungen enthaltend TMM1, V1, M1, M3, M6 und TEG1**

Lösungen, wie sie in Tabelle 2 zusammengefasst sind, werden wie folgt hergestellt: Zunächst werden 250 mg der Kompositionen in 10 ml Chlorbenzol gelöst und so lange gerührt, bis die Lösung klar ist. Die Lösung wird unter Verwendung eines Filters (Millipore Millex LS, Hydrophobic PTFE 5.0 μm) filtriert.

**Tabelle 2: Zusammensetzung der Lösungen**

| Lösung | Zusammensetzung | Verhältnis (bezogen auf Gewicht) | Lösemittel | Konzentration |
|---|---|---|---|---|
| 1 | TMM1 + M1 + TEG1 | 45:45:10 | Chlorbenzol | 25 mg/ml |
| 2 | TMM1 + M3 + TEG1 | 45:45:10 | Chlorbenzol | 25 mg/ml |
| 3 | M3+ TEG1 | 80:20 | Chlorbenzol | 25 mg/ml |
| 4 | TMM1 + M6 + TEG1 | 45:45:10 | Chlorbenzol | 25 mg/ml |
| 5 | TMM1 + V1 + TEG1 | 45:45:10 | Chlorbenzol | 25 mg/ml |
| 6 | M7 | 100% | Chlorbenzol | 20 mg/ml |

Die Lösungen 1 bis 5 werden verwendet, um die emittierende Schicht von OLEDs zu beschichten. Die entsprechende Feststoffzusammensetzung kann erhalten werden, indem das Lösemittel der Lösungen verdampft wird. Diese kann für die Herstellung weiterer Formulierungen verwendet werden.

**Beispiel 8**

**Herstellung der OLEDs**

OLED1 bis OLED5 mit einer Struktur gemäß dem Stand der Technik, Anode(ITO)/PEDOT/Interlayer/EML/Kathode (EML = Emissionslayer; ITO = Indiumzinnoxid), werden unter Verwendung der entsprechenden Lösungen 1 bis 5, wie in Tabelle 2 zusammengefasst, gemäß der folgenden Vorschrift hergestellt:

1. Beschichtung von 80 nm PEDOT (Baytron P AI 4083) auf ein ITObeschichtetes Glassubstrat durch Spin-Coating.

2. Beschichtung eines 20 nm Interlayers durch Spin-Coating einer Toluollösung von HIL-012 (Konzentration 0.5 Gew.%) in einer Glovebox.

3. Ausheizen der Interlayer bei 180°C für 1 h in einer Glovebox.

4. Beschichtung einer 80 nm emittierenden Schicht (EML) durch Spin-Coating einer Lösung gemäß Tabelle 2.

5. Ausheizen der Vorrichtung bei 120°C für 20 min.

6. Aufdampfen einer Ba/Al-Kathode (3 nm + 150 nm).

7. Verkapselung der Vorrichtung.

**Beispiel 8**

**Messungen den OLEDs und Vergleich der Ergebnisse**

Die so erhaltenen OLEDs werden nach Standardmethoden charakterisiert. Dabei werden die folgenden Eigenschaften gemessen: UIL-Charakteristik, Elektrolumineszenzspektrum, Farbkoordinaten, Effizienz, Betriebsspannung und Lebensdauer. Die Ergebnisse sind in Tabelle 3 zusammengefasst, wobei OLED5 als Vergleich gemäß dem Stand der Technik dient. In Tabelle 3 steht $U_{on}$ für die Einsatzspannung, U(100) für die Spannung bei 100 cd/$m^2$ und U(1000) für die Spannung bei 1000 cd/$m^2$.

**Tabelle 3:** Messergebnisse mit OLED1 bis OLED5

| | Max. Eff. [cd/A] | $U_{on}$ [V] | U(100) [V] | U(1000) [V] | CIE @ 100 cd/$m^2$ |
|---|---|---|---|---|---|
| OLED1* | 27.2 | 2.80 | 3.78 | 6.10 | 0.34 / 0.62 |
| OLED2 | 29.3 | 2.78 | 3.90 | 5.38 | 0.35/0.61 |
| OLED3 | 25.3 | 2.84 | 4.10 | 5.97 | 0.35/ 0.62 |
| OLED4 | 28.1 | 2.73 | 3.61 | 5.65 | 0.34 / 0.62 |
| OLED5 (Vergleich) | 18.3 | 3.60 | 5.70 | 7.40 | 0.35/0.61 |
| *nicht erfindungsgemäß | | | | | |

Wie aus Tabelle 3 ersichtlich ist, zeigen die erfindungsgemäßen organischen Elektrolumineszenzvorrichtungen mit M3 und M6 als Co-Matrixmaterialien oder Matrixmaterial deutlich verbesserte phosphoreszierende OLEDs in Bezug auf Betriebsspannung und Effizienz erhalten. Es kann daran liegen, dass M3 und M6 hohe T1 Level haben, und auch ein günstiges HOMO Level haben, sodass es einen besseren Lochtransport ermöglichen kann. Alle OLEDs zeigen vergleichbare Farbkoordinaten.

**Beispiel 9**

**OFET basierende auf M7 (nicht erfindungsgemäß)**

Dünnschicht-Bottom-Gate organischen Feldeffekt-Transistoren (OFETs) sind in einer trockenen Stickstoffatmosphäre Glovebox auf hochdotierten Silizium-Substrate gefertigt, mit thermisch gewachsenen Siliziumoxid ($SiO_2$)

Isolierschicht (Dicke 230 nm), wobei das Substrat als gemeinsame Gate-Elektrode dient. Transistor-Source-Drain-Gold-Kontakte sind photolithographisch auf der $SiO_2$-Schicht definiert. FET Substrate sind Lösungsmittel gereinigt und anschließend Ozon behandelt für 10 min. in einem speziell angefertigten Quecksilber-Niederdruck-Lampe-Setup. Die Devices werden dann so behandelt, zuerst mit Octyltrichlorsilan durch Eintauchen des Substrats in 10 mM Lösungen in Toluol (erhitzt bei 60°C) für 15 min., und dann ein gründliches Waschen mit Hexan, Aceton und Isopropanol. Die dünnen Halbleiterschichten werden anschließend durch Spin-Coating der Lösung 6 bei einer Umdrehungsgeschwindigkeit von ca. 3.000 U/min aufgebracht. Die Devices werden dann getrocknet und ausgeheizt bei 100°C für 10 min. und unter Ausschluss von Licht gemessen. Feldeffekt-Mobilität $\mu^{sat}$ wird in dem Sättigungsregime ($V_d$ > ($V_g$-$V_0$)) unter Verwendung von Gleichung (1) berechnet:

$$\left(\frac{dI_d^{sat}}{dV_g}\right)_{V_d} = \frac{WC_i}{L}\mu^{sat}\left(V_g - V_0\right)$$

Gleichung (1)

wobei W die Kanalbreite, L die Kanallänge, Ci die Kapazität der Isolierschicht, $V_d$ die Drain-Spannung, $V_g$ die Gate-Spannung, $V_0$ die Schaltspannung und $I_d$ der Drain-Strom ist.

Die OFETs zeigen eine Mobilität von 0.005 $cm^2/Vs$, und eine On/Off Ratio von $3x10^5$.

**Patentansprüche**

1. Organische elektronische Vorrichtung, **dadurch gekennzeichnet, dass** sie in wenigstens einer Schicht eine Verbindung gemäß der folgenden Formel (9) enthält:

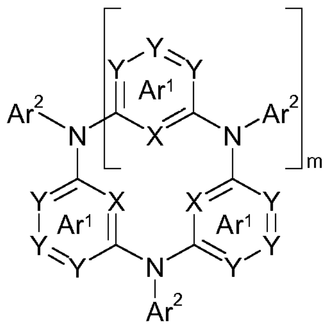

Formel (9),

wobei die verwendeten Symbole und Indizes die folgenden Bedeutungen aufweisen:

$Ar^2$ ist bei jedem Auftreten gleich oder verschieden ein substituiertes oder unsubstituiertes monovalentes monocyclisches oder polycyclisches aromatisches oder heteroaromatisches Ringsystem;
X und Y sind gleich $CR^1$ oder N;
m ist eine ganze Zahl von 1 bis 6;
mit der Maßgabe, dass entweder 1.) mindestens ein Vertreter der X oder Y gleich N ist, oder 2.) ein Vertreter der $Ar^2$ ein heteroaromatisches Ringsystem ist;

und wobei

$R_1$ bei jedem Auftreten gleich oder verschieden aus der Gruppe ausgewählt ist, die aus folgendem besteht: H, D, Halogen, CHO, $N(Ar)_2$, C(=O)Ar, $P(=O)(Ar)_2$, S(=O)Ar, $S(=O)_2Ar$, $CR^2=CR^2Ar$, CN, $NO_2$, $Si(R^2)_3$, $B(OR^2)_2$, $B(R^2)_2$, $B(N(R^2)_2)_2$, $OSO_2R^2$, eine Alkyl-, Alkoxy- oder Thioalkoxygruppe, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch F, Cl, Br, I, CN oder $NO_2$ ersetzt sein können, oder

ein mono- oder polycylisches aromatisches oder heteroaromatisches Ringsystem, das jeweils durch einen oder mehrere Reste $R^2$ substituiert sein kann, oder eine Aryloxy- oder Heteroaryloxygruppe, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches oder aromatisches Ringsystem bilden;

Ar ist bei jedem Auftreten gleich oder verschieden ein aromatisches oder heteroaromatisches Ringsystem, das mit einem oder mehreren Resten $R^2$ substituiert sein kann; dabei können auch zwei Reste Ar, welche an dasselbe Stickstoff-, Phosphor- oder Boratom binden, durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verknüpft sein;

$R^2$ ist bei jedem Auftreten gleich oder verschieden H, D oder ein aliphatischer, aromatischer und/oder heteroaromatischer Kohlenwasserstoffrest, in dem auch H-Atome durch F ersetzt sein können; dabei können zwei oder mehrere benachbarte Substituenten $R^2$ auch miteinander ein mono- oder polycyclisches, aliphatisches oder aromatisches Ringsystem bilden.

2. Organische elektronische Vorrichtung nach Anspruch 1, **dadurch gekennzeichnet, dass** die wenigstens eine Schicht eine Elektronentransportsschicht, Elektroneninjektionsschicht, Lochblockierschicht, Exzitonenblockierschicht oder eine emittierende Schicht, bevorzugt eine emittierende Schicht ist.

3. Organische elektronische Vorrichtung nach Anspruch 1 oder 2, **dadurch gekennzeichnet, dass** in der Verbindung nach Formel (9) wenigstens ein Vertreter der $Ar^1$ und/oder $Ar^2$ ein heteroaromatisches Ringsystem darstellt.

4. Organische elektronische Vorrichtung nach einem oder mehreren der Ansprüche 1 bis 3, **dadurch gekennzeichnet, dass** wenigstens ein Vertreter der $Ar^1$ eine bivalente Gruppe gemäß einer der folgenden Formeln (2) bis (8) ist:

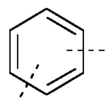

Formel (2)

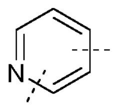

Formel (3)

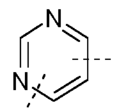

Formel (4)

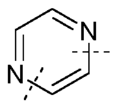

Formel (5)

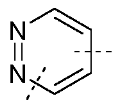

Formel (6)

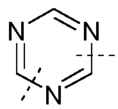

Formel (7)

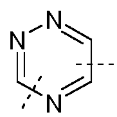

Formel (8),

wobei die gestrichelten Linien die Bindungen zu den Ringstickstoffatomen in Formel (9) darstellen und diese Bindungen über Kohlenstoffatome in den Positionen 1 und 3 der Verbindungen der Formeln (2) bis (8) geknüpft sind.

5. Organische elektronische Vorrichtung nach Anspruch 1, **dadurch gekennzeichnet, dass** die wenigstens eine Schicht eine Lochtransportschicht oder Lochinjektionsschicht ist, und wobei X und Y gleich $CR^1$ oder N und m eine ganze Zahl von 1 bis 6 ist, mit der Maßgabe, dass mindestens ein Vertreter der X oder Y gleich N ist.

6. Organische elektronische Vorrichtung nach einem oder mehreren der Ansprüche 1 bis 5, **dadurch gekennzeichnet, dass** wenigstens ein $Ar^2$ ein monovalenter Rest ist, der aus der Gruppe ausgewählt ist, die aus den Verbindungen der folgenden Formeln (144) bis (156):

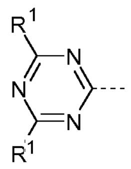
Formel (144)

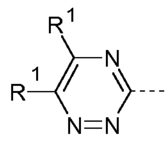
Formel (145)

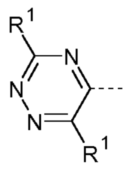
Formel (146)

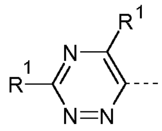
Formel (147)

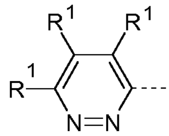
Formel (148)

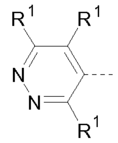
Formel (149)

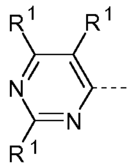
Formel (150)

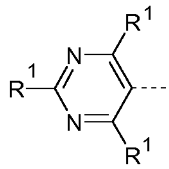
Formel (151)

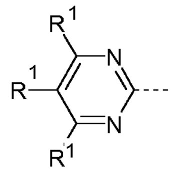
Formel (152)

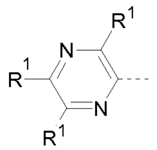
Formel (153)

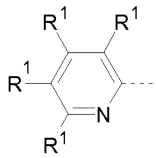
Formel (154)

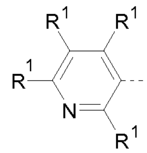
Formel (155)

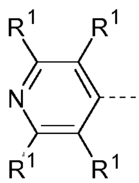
Formel (156),

wobei
die gestrichelte Linie jeweils eine Bindung zum Ringstickstoffatom der Verbindung der Formel (9) darstellt; und die Reste $R^1$ die gleiche Bedeutung wie in Anspruch 1 definiert haben.

7. Organische elektronische Vorrichtung nach einem oder mehreren der Ansprüche 1 bis 4 und 6, **dadurch gekennzeichnet, dass** die wenigstens eine Schicht eine emittierende Schicht ist, die zusätzlich eine phosphoreszierende Emitterverbindung enthält.

8. Organische elektronische Vorrichtung nach Anspruch 7, worin die phosphoreszierende Emitterverbindung eine Verbindung ist, die Kupfer, Molybdän, Wolfram, Rhenium, Ruthenium, Osmium, Rhodium, Iridium, Palladium, Platin, Silber, Gold oder Europium enthält. insbesondere eine Verbindung, die Iridium oder Platin enthält.

9. Organische elektronische Vorrichtung nach einem oder mehreren der Ansprüche 1 bis 4 und 6, **dadurch gekennzeichnet, dass** die wenigstens eine Schicht eine ETL oder EIL ist, die zusätzlich eine phosphoreszierende Emitterverbindung enthält.

**10.** Organische elektronische Vorrichtung nach einem oder mehreren der Ansprüche 1 bis 9, worin die Vorrichtung eine organische Elektrolumineszenzvorrichtung ist.

**11.** Organische elektronische Vorrichtung nach Anspruch 10, die eine OLED, OLEC, ein "organic light emitting field effect transistor" oder eine "organic light emitting electrochemical cell" ist.

**12.** Verwendung einer Verbindung gemäß Formel (9), wie in einem oder mehreren der Ansprüche 1 bis 4 und 6 definiert, als Matrixmaterialien für phosphoreszierende Emitterverbindungen oder als Elektronentransportmaterialien in einer organischen elektronischen Vorrichtung.

**13.** Zusammensetzung enthaltend mindestens eine Verbindung gemäß Formel (9), wie in einem oder mehreren der Ansprüche 1 bis 4 und 6 definiert, und mindestens eine phosphoreszierende Emitterverbindung.

**14.** Verbindung der Formel (9) wie in Anspruch 1 definiert, mit der Maßgabe, dass wenigstens ein Vertreter der Y gleich N und wenigstens ein Vertreter der X gleich $CR^1$ ist.

**15.** Verwendung einer Verbindung nach Anspruch 14 in einer organischen elektronischen Vorrichtung.

**16.** Organische elektronische Vorrichtung enthaltend mindestens eine Verbindung nach Anspruch 14, bevorzugt ausgewählt aus der Gruppe bestehend aus organischen Elektrolumineszenzvorrichtungen (organischen Leuchtdioden (OLEDs), organischen lichtemittierenden Transistoren (O-LETs), organisch lichtemittierenden elektrochemischen Zellen (OLECs) und organisch lichtemittierenden elektrochemischen Transistoren), organischen integrierten Schaltungen (O-ICs), organischen Feld-Effekt-Transistoren (O-FETs), organischen Dünnfilmtransistoren (O-TFTs), organischen Solarzellen (O-SCs), farbstoffsensibilisierten organischen Solarzellen (ODSSCs), organischen optischen Detektoren, organischen Photorezeptoren, organischen Feld-Quench-Devices (O-FQDs), organischen Laserdioden (O-Laser) und organic plasmon emitting devices.

**17.** Organische Elektrolumineszenzvorrichtung nach Anspruch 16, **dadurch gekennzeichnet, dass** die Verbindung nach Anspruch 14 als Matrixmaterial für fluoreszierende oder phosphoreszierende Emitter und/oder in einer Lochblockierschicht und/oder in einer Elektronentransportschicht und/oder in einer elektronenblockierenden bzw. exzitonenblockierenden Schicht und/oder in einer Lochtransportschicht verwendet werden.

**18.** Formulierung, insbesondere eine Lösung, Dispersion oder Mini-emulsion, enthaltend mindestens eine Verbindung nach Anspruch 14 und mindestens ein Lösungsmittel.

**19.** Mischung enthaltend mindestens eine Verbindung nach Anspruch 14 und mindestens eine weitere Verbindung.

## Claims

**1.** Organic electronic device, **characterised in that** it comprises, in at least one layer, a compound of the following formula (9):

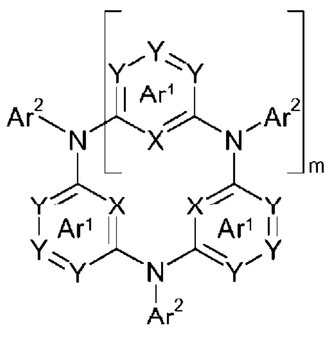

formula (9)

where the symbols and indices used have the following meanings:

$Ar^2$ is on each occurrence, identically or differently, a substituted or unsubstituted monovalent monocyclic or polycyclic aromatic or heteroaromatic ring system;
X and Y are equal to $CR^1$ or N;
m is an integer from 1 to 6;
with the proviso that either 1.) at least one representative of the X or Y is equal to N, or 2.) one representative of the $Ar^2$ is a heteroaromatic ring system;

and where

$R^1$ is selected on each occurrence, identically or differently, from the group consisting of the following: H, D, halogen, CHO, $N(Ar)_2$, C(=O)Ar, $P(=O)(Ar)_2$, S(=O)Ar, $S(=O)_2Ar$, $CR^2$=$CR^2Ar$, CN, $NO_2$, $Si(R^2)_3$, $B(OR^2)_2$, $B(R^2)_2$, $B(N(R^2)_2)_2$, $OSO_2R^2$, an alkyl, alkoxy or thioalkoxy group, which may in each case be substituted by one or more radicals $R^2$, where one or more non-adjacent $CH_2$ groups may be replaced by $R^2C$=$CR^2$, C≡C, $Si(R^2)_2$, $Ge(R^2)_2$, $Sn(R^2)_2$, C=O, C=S, C=Se, C=$NR^2$, P(=O)($R^2$), SO, $SO_2$, $NR^2$, O, S or $CONR^2$ and where one or more H atoms may be replaced by F, Cl, Br, I, CN or $NO_2$, or a mono- or polycyclic aromatic or heteroaromatic ring system, which may in each case be substituted by one or more radicals $R^2$, or an aryloxy or heteroaryloxy group, which may be substituted by one or more radicals $R^2$, or a combination of these systems; two or more adjacent substituents $R^1$ may also form a mono- or polycyclic, aliphatic or aromatic ring system with one another;
Ar is on each occurrence, identically or differently, an aromatic or heteroaromatic ring system, which may be substituted by one or more radicals $R^2$; two radicals Ar which are bonded to the same nitrogen, phosphorus or boron atom may also be linked to one another by a single bond or a bridge selected from B($R^2$), $C(R^2)_2$, $Si(R^2)_2$, C=O, C=$NR^2$, C=$C(R^2)_2$, O, S, S=O, $SO_2$, N($R^2$), P($R^2$) and P(=O)$R^2$;
$R^2$ is on each occurrence, identically or differently, H, D or an aliphatic, aromatic and/or heteroaromatic hydrocarbon radical, in which, in addition, H atoms may be replaced by F; two or more adjacent substituents $R^2$ may also form a mono- or polycyclic, aliphatic or aromatic ring system with one another.

2. Organic electronic device according to Claim 1, **characterised in that** the at least one layer is an electron-transport layer, electron-injection layer, hole-blocking layer, exciton-blocking layer or an emitting layer, preferably an emitting layer.

3. Organic electronic device according to Claim 1 or 2, **characterised in that** at least one representative of the $Ar^1$ and/or $Ar^2$ in the compound of the formula (9) represents a heteroaromatic ring system.

4. Organic electronic device according to one or more of Claims 1 to 3, **characterised in that** at least one representative of the $Ar^1$ is a divalent group of one of the following formulae (2) to (8):

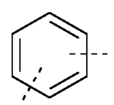
formula (2)

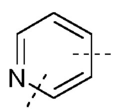
formula (3)

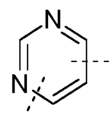
formula (4)

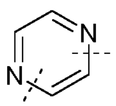
formula (5)

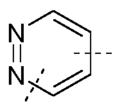
formula (6)

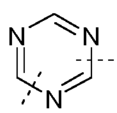
formula (7)

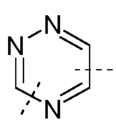
formula (8)

where the dashed lines represent the bonds to the ring nitrogen atoms in formula (9) and these bonds are linked via carbon atoms in positions 1 and 3 of the compounds of the formulae (2) to (8).

5. Organic electronic device according to Claim 1, **characterised in that** the at least one layer is a hole-transport layer or hole-injection layer, and where X and Y are equal to $CR^1$ or N and m is an integer from 1 to 6, with the proviso that at least one representative of the X or Y is equal to N

6. Organic electronic device according to one or more of Claims 1 to 5, **characterised in that** at least one $Ar^2$ is a monovalent radical selected from the group consisting of the compounds of the following formulae (144) to (156):

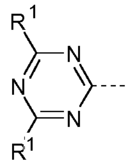

formula (144)

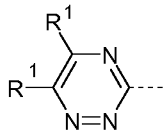

formula (145)

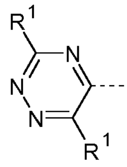

formula (146)

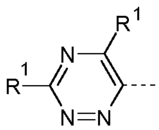

formula (147)

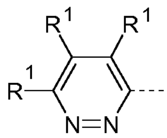

formula (148)

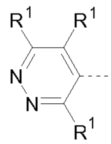

formula (149)

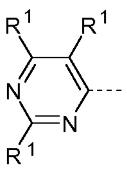

formula (150)

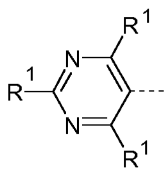

formula (151)

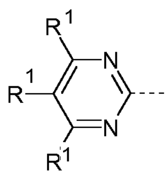

formula (152)

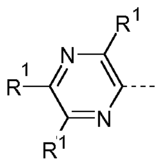

formula (153)

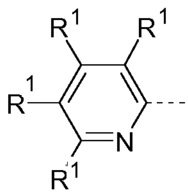

formula (154)

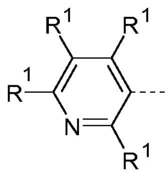

formula (155)

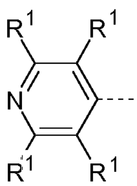

formula (156)

where
the dashed line in each case represents a bond to the ring nitrogen atom of the compound of the formula (9); and the radicals $R^1$ have the same meaning as defined in Claim 1.

7. Organic electronic device according to one or more of Claims 1 to 4 and 6, **characterised in that** the at least one layer is an emitting layer which additionally comprises a phosphorescent emitter compound.

8. Organic electronic device according to Claim 7, in which the phosphorescent emitter compound is a compound which contains copper, molybdenum, tungsten, rhenium, ruthenium, osmium, rhodium, iridium, palladium, platinum, silver, gold or europium, in particular a compound which contains iridium or platinum.

9. Organic electronic device according to one or more of Claims 1 to 4 and 6, **characterised in that** the at least one layer is an ETL or EIL which additionally comprises a phosphorescent emitter compound.

10. Organic electronic device according to one or more of Claims 1 to 9, in which the device is an organic electroluminescent device.

11. Organic electronic device according to Claim 10, which is an OLED, OLEC, an organic light-emitting field effect transistor or an organic light-emitting electrochemical cell.

12. Use of a compound of the formula (9), as defined in one or more of Claims 1 to 4 and 6, as matrix materials for phosphorescent emitter compounds or as electron-transport materials in an organic electronic device.

13. Composition comprising at least one compound of the formula (9), as defined in one or more of Claims 1 to 4 and 6, and at least one phosphorescent emitter compound.

14. Compound of the formula (9) as defined in Claim 1, with the proviso that at least one representative of the Y is equal to N and at least one representative of the X is equal to $CR^1$.

15. Use of a compound according to Claim 14 in an organic electronic device.

16. Organic electronic device containing at least one compound according to Claim 14, preferably selected from the group consisting of organic electroluminescent devices (organic light-emitting diodes, OLEDs), organic light-emitting transistors (O-LETs), organic light-emitting electrochemical cells (OLECs) and organic light-emitting electrochemical transistors), organic integrated circuits (O-ICs), organic field-effect transistors (O-FETs), organic thin-film transistors (O-TFTs), organic solar cells (O-SCs), dye-sensitised organic solar cells (ODSSCs), organic optical detectors, organic photoreceptors, organic field-quench devices (O-FQDs), organic laser diodes (O-lasers) and organic plasmon emitting devices.

17. Organic electroluminescent device according to Claim 16, **characterised in that** the compound according to Claim 14 is used as matrix material for fluorescent or phosphorescent emitters and/or in a hole-blocking layer and/or in an electron-transport layer and/or in an electron-blocking or exciton-blocking layer and/or in a hole-transport layer.

18. Formulation, in particular a solution, dispersion or mini-emulsion, comprising at least one compound according to Claim 14, and at least one solvent.

19. Mixture comprising at least one compound according to Claim 14 and at least one further compound.

## Revendications

1. Dispositif électronique organique, **caractérisé en ce qu'**il comprend, dans au moins une couche, un composé de la formule (9) qui suit :

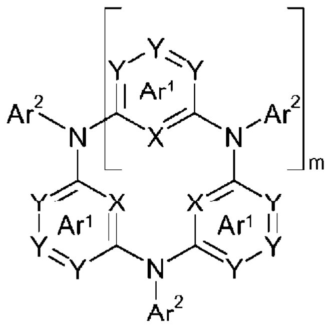

formule (9)

dans laquelle les symboles et les indices qui sont utilisés présentent les significations qui suivent :

$Ar^2$ est pour chaque occurrence, de manière identique ou différente, un système de cycle aromatique ou hétéroaromatique monocyclique ou polycyclique monovalent substitué ou non substitué ;
X et Y sont égaux à $CR^1$ ou N ;
m est un entier de 1 à 6 ;

étant entendu que soit 1.) au moins l'un représentatif de X ou Y est égal à N, soit 2.) l'un représentatif de $Ar^2$ est un système de cycle hétéroaromatique ;
et dans laquelle :

$R^1$ est sélectionné pour chaque occurrence, de manière identique ou différente, parmi le groupe qui est constitué par ce qui suit : H, D, halogène, CHO, $N(Ar)_2$, C(=O)Ar, $P(=O)(Ar)_2$, S(=O)Ar, $S(=O)_2Ar$, $CR^2=CR^2Ar$, CN, $NO_2$, $Si(R^2)_3$, $B(OR^2)_2$, $B(R^2)_2$, $B(N(R^2)_2)_2$, $OSO_2R^2$, un groupe alkyle, alcoxy ou thioalcoxy, lequel peut dans chaque cas être substitué par un radical ou par plusieurs radicaux $R^2$, où un ou plusieurs groupe(s) $CH_2$ non adjacents peut/peuvent être remplacé(s) par $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par F, Cl, Br, I, CN ou $NO_2$, ou un système de cycle aromatique ou hétéroaromatique mono- ou polycyclique, lequel peut dans chaque cas être substitué par un radical ou par plusieurs radicaux $R^2$, ou un groupe aryloxy ou hétéroaryloxy, lequel peut être substitué par un radical ou par plusieurs radicaux $R^2$, ou une combinaison de ces systèmes ; deux substituants $R^1$ adjacents ou plus peuvent également former un système de cycle aliphatique ou aromatique mono- ou polycyclique l'un avec l'autre ou les uns avec les autres ;
Ar est pour chaque occurrence, de manière identique ou différente, un système de cycle aromatique ou hétéroaromatique, lequel peut être substitué par un radical ou par plusieurs radicaux $R^2$; deux radicaux Ar qui sont liés au même atome d'azote, de phosphore ou de bore peuvent également être liés l'un à l'autre par une liaison simple ou un pont sélectionné parmi $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ et $P(=O)R^2$ ;
$R^2$ est pour chaque occurrence, de manière identique ou différente, H, D ou un radical hydrocarbone aliphatique, aromatique et/ou hétéroaromatique, où, en outre, des atomes de H peuvent être remplacés par F ; deux substituants $R^2$ adjacents ou plus peuvent également former un système de cycle aliphatique ou aromatique mono- ou polycyclique l'un avec l'autre ou les uns avec les autres.

**2.** Dispositif électronique organique selon la revendication 1, **caractérisé en ce que** l'au moins une couche est une couche de transport d'électrons, une couche d'injection d'électrons, une couche de blocage de trous, une couche de blocage d'excitons ou une couche d'émission, de préférence une couche d'émission.

**3.** Dispositif électronique organique selon la revendication 1 ou 2, **caractérisé en ce qu'**au moins l'un représentatif de $Ar^1$ et/ou $Ar^2$ dans le composé de la formule (9) représente un système de cycle hétéroaromatique.

**4.** Dispositif électronique organique selon une ou plusieurs des revendications 1 à 3, **caractérisé en ce qu'**au moins l'un représentatif de $Ar^1$ est un groupe divalent de l'une des formules (2) à (8) qui suivent :

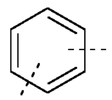
formule (2)

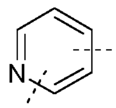
formule (3)

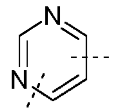
formule (4)

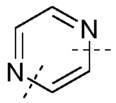
formule (5)

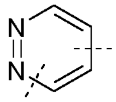
formule (6)

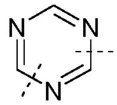
formule (7)

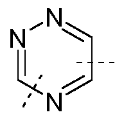
formule (8)

dans lesquelles les lignes en pointillés représentent les liaisons sur les atomes d'azote de cycle dans la formule (9) et ces liaisons sont liées via des atomes de carbone au niveau des positions 1 et 3 des composés des formules (2) à (8).

**5.** Dispositif électronique organique selon la revendication 1, **caractérisé en ce que** l'au moins une couche est une couche de transport de trous ou une couche d'injection de trous, et où X et Y sont égaux à $CR^1$ ou N et m est un entier de 1 à 6, étant entendu qu'au moins l'un représentatif de X ou Y est égal à N.

**6.** Dispositif électronique organique selon une ou plusieurs des revendications 1 à 5, **caractérisé en ce qu'**au moins un $Ar^2$ est un radical monovalent qui est sélectionné parmi le groupe qui est constitué par les composés des formules (144) à (156) qui suivent :

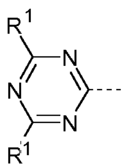
formule (144)

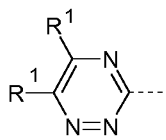
formule (145)

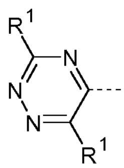
formule (146)

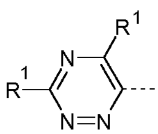
formule (147)

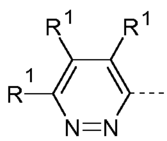
formule (148)

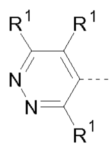
formule (149)

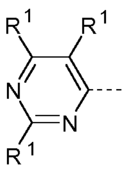
formule (150)

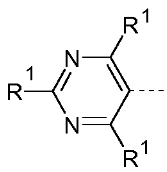
formule (151)

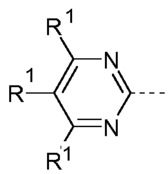
formule (152)

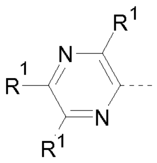

formule (153)

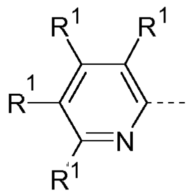

formule (154)

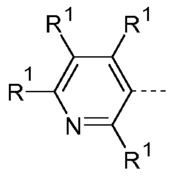

formule (155)

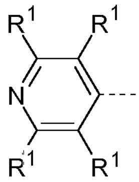

formule (156)

dans lesquelles :
la ligne en pointillés représente dans chaque cas une liaison sur l'atome d'azote de cycle du composé de la formule (9) ; et les radicaux $R^1$ présentent la même signification que définie selon la revendication 1.

**7.** Dispositif électronique organique selon une ou plusieurs des revendications 1 à 4 et 6, **caractérisé en ce que** l'au moins une couche est une couche d'émission qui comprend de façon additionnelle un composé d'émetteur phosphorescent.

**8.** Dispositif électronique organique selon la revendication 7, dans lequel le composé d'émetteur phosphorescent est un composé qui contient du cuivre, du molybdène, du tungstène, du rhénium, du ruthénium, de l'osmium, du rhodium, de l'iridium, du palladium, du platine, de l'argent, de l'or ou de l'europium, en particulier un composé qui contient de l'iridium ou du platine.

**9.** Dispositif électronique organique selon une ou plusieurs des revendications 1 à 4 et 6, **caractérisé en ce que** l'au moins une couche est une ETL ou une EIL, laquelle comprend de façon additionnelle un composé d'émetteur phosphorescent.

**10.** Dispositif électronique organique selon une ou plusieurs des revendications 1 à 9, dans lequel le dispositif est un dispositif électroluminescent organique.

**11.** Dispositif électronique organique selon la revendication 10, lequel est une OLED, une OLEC, un transistor à effet de champ à émission de lumière organique ou une cellule électrochimique à émission de lumière organique.

**12.** Utilisation d'un composé de la formule (9), tel que défini selon une ou plusieurs des revendications 1 à 4 et 6, en tant que matériaux de matrices pour des composés d'émetteur phosphorescents ou en tant que matériaux de transport d'électrons dans un dispositif électronique organique.

**13.** Composition comprenant au moins un composé de la formule (9), tel que défini selon une ou plusieurs des revendications 1 à 4 et 6, et au moins un composé d'émetteur phosphorescent.

**14.** Composé de la formule (9) tel que défini selon la revendication 1, étant entendu qu'au moins l'un représentatif de Y est égal à N et au moins l'un représentatif de X est égal à $CR^1$.

**15.** Utilisation d'un composé selon la revendication 14 dans un dispositif électronique organique.

**16.** Dispositif électronique organique contenant au moins un composé selon la revendication 14, de préférence sélectionné parmi le groupe qui est constitué par les dispositifs électroluminescents organiques (les diodes émettrices de lumière organiques, OLED), les transistors à émission de lumière organiques (O-LET), les cellules électrochimiques à émission de lumière organiques (OLEC) et les transistors électrochimiques à émission de lumière organiques), les circuits intégrés organiques (O-IC), les transistors à effet de champ organiques (O-FET), les transistors à film mince organiques (O-TFT), les cellules solaires organiques (O-SC), les cellules solaires organiques sensibilisées par colorant(s) (ODSSC), les détecteurs optiques organiques, les photorécepteurs organiques, les dispositifs à extinction de champ organiques (O-FQD), les diodes laser organiques (O-laser) et les dispositifs à émission de plasmons organiques .

**17.** Dispositif électroluminescent organique selon la revendication 16, **caractérisé en ce que** le composé selon la revendication 14 est utilisé en tant que matériau de matrice pour des émetteurs fluorescents ou phosphorescents et/ou dans une couche de blocage de trous et/ou dans une couche de transport d'électrons et/ou dans une couche de blocage d'électrons ou une couche de blocage d'excitons et/ou dans une couche de transport de trous.

**18.** Formulation, en particulier une solution, une dispersion ou une mini-émulsion, comprenant au moins un composé selon la revendication 14, et au moins un solvant

**19.** Mélange comprenant au moins un composé selon la revendication 14 et au moins un autre composé.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- WO 2004093207 A
- WO 2005003253 A
- WO 2007063754 A
- WO 2008056746 A
- US 2002058155 A1
- JP 2007214364 A
- WO 2008082164 A1
- US 2002045061 A1
- WO 2005011013 A
- WO 007065 A
- WO 0141512 A
- WO 0202714 A
- WO 0215645 A
- EP 1191613 A
- EP 1191612 A
- EP 1191614 A
- WO 2005033244 A
- WO 0070655 A
- WO 2007050301 A
- WO 2007050334 A
- EP 1144543 A
- WO 2005039246 A
- US 20050069729 A
- JP 2004288381 A
- EP 1205527 A
- WO 08086851 A
- EP 1617710 A
- EP 1617711 A
- EP 1731584 A
- JP 2005347160 A
- WO 2007137725 A
- DE 102008017591
- WO 07137725 A
- WO 05111172 A
- WO 06117052 A
- WO 02072714 A
- WO 2003019694 A
- WO 2008086851 A
- WO 2004026886 A


### In der Beschreibung aufgeführte Nicht-Patentliteratur


- **M. A. BALDO et al.** *Appl. Phys. Lett.,* 1999, vol. 75, 4-6
- **Y. HINO et al.** *Jpn. J. Appl. Phys.,* 2005, vol. 44, 2790-2794
- **R. FRIEND et al.** Organic light emitting diode'' (OLED). *Nature,* 1999, vol. 397, 121-128
- **PEI et al.** Organic light emitting electrochemical cell'' (OLEC). *Science,* 1995, vol. 269, 1086
- **FABIO ; CICOIRA ; CLARA SANTATO.** Organic light emitting field effect transistor. *Adv. Funct. Mater.,* 2007, vol. 17, 3421-3434
- **C. YUMUSAK ; N. S. SARICIFTCI.** Organic light emitting electrochemical transistor. *Appl. Phys. Lett.,* 2010, vol. 97, 033302
- **T. MATSUMOTO ; T. NAKADA ; J. ENDO ; K. MORI ; N. KAWAMURA ; A. YOKOI ; J. KIDO.** *Multiphoton Organic EL Device Having Charge Generation Layer*
- **M. S. ARNOLD et al.** *Appl. Phys. Lett.,* 2008, vol. 92, 053301
- **D. M. KOLLER et al.** organic plasmon emitting devices. *Nature Photonics,* 2008, 1-4